# EUROPEAN PATENT SPECIFICATION

(11) **EP 4 504 933 B1**
(45) Date of publication and mention of the grant of the patent: **03.06.2026**
(21) Application number: 23716918.0
(22) Date of filing: 04.04.2023
(51) Int. Cl.: C12N 15/10, C12Q 1/6806

(54) **METHOD**
VERFAHREN
PROCÉDÉ

(30) Priority: 04.04.2022 GB 202204919
(43) Date of publication of application: 12.02.2025
(73) Proprietor: Oxford Nanopore Technologies PLC, Oxford OX4 4DQ (GB)
(72) Inventor: STODDART, David Jackson, Oxford Oxfordshire OX4 4DQ (GB); TURNER, Daniel John, Oxford Oxfordshire OX4 4DQ (GB)
(74) Representative: J A Kemp LLP
(86) International application number: PCT/GB2023/050889
(87) International publication number: WO 2023/194713

(56) References cited:
- EP-A1- 3 527 672

## Description

### Field

The disclosure relates to methods of attaching a nucleic acid adapter to a target polynucleotide, and methods of preparing a nucleic acid library, using a topoisomerase activated adapter. The disclosure also relates to methods of producing adapted PCR amplicons, and kits for carrying out the methods of the disclosure.

### Background

There is currently a need for rapid and cheap polynucleotide (e.g. DNA or RNA) sequencing and identification technologies across a wide range of applications. Existing technologies are slow and expensive mainly because they rely on amplification techniques to produce large volumes of polynucleotide and require a high quantity of specialist fluorescent chemicals for signal detection.

Transmembrane pores (nanopores) have great potential as direct, electrical biosensors for polymers and a variety of small molecules. In particular, recent focus has been given to nanopores as a potential DNA sequencing technology.

When a potential is applied across a nanopore, there is a change in the current flow when an analyte, such as a nucleotide, resides transiently in the barrel for a certain period of time. Nanopore detection of the nucleotide gives a current change of known signature and duration. In the strand sequencing method, a single polynucleotide strand is passed through the pore and the identities of the nucleotides are derived. Strand sequencing can involve the use of a molecular brake to control the movement of the polynucleotide through the pore.

There are many commercial situations, including polynucleotide sequencing and identification technologies, which require the attachment of an adapter to a target polynucleotide and the preparation of a nucleic acid library. This may be achieved using topoisomerase activated adapters.

Cheng & Shuman (Nucleic Acids Research, 2000, Vol. 28, No. 9, 1893-1898) describe DNA strand transfer catalysed by vaccinia topoisomerase and ligation of DNAs containing a 3' mononucleotide overhang.

US 2002/0068290 describes topoisomerase activated nucleotide adapters, and methods and compositions for the rapid joining of a target nucleic acid sequence with a topoisomerase activated adapter sequence that provides a specific function to the target.

US 2016/0348152 describes compositions comprising activated topoisomerase adapters and methods of using the activated topoisomerase adapters for preparing a library of target DNA duplexes derived from sample polynucleotides, such as for use in Next Generation Sequencing methods.

WO 2016/059436 describes a method of attaching a double stranded DNA onto a strand of RNA using a topoisomerase, for use in nanopore sequencing. EP3527672 discloses the use of a topoisomerase for the preparation of sequencing libraries.

### Summary

The present inventors have identified a novel method for attaching a nucleic acid adapter to a double stranded target polynucleotide together with a novel method of preparing a library of nucleic acids. Particularly, the inventors have determined that the disclosed methods utilising topoisomerase-activated sequencing adapters can covalently attach to some amplicon 5' ends more readily than to others, thus advantageously enabling improved methods of creating nucleic acid libraries.

The methods involve the use of vaccinia topoisomerase I activated adapters to covalently attach a nucleic acid adapter to the 5' end of a double stranded target polynucleotide comprising a triplet nucleotide sequence at the 5' end of one or both strands, wherein the triplet nucleotide sequence is selected from GGT, GGG, TGT, GTT, TGG, GGA, GTG, CGG, CGT and GCG. The inventors have shown that vaccinia topoisomerase I-activated adapters can covalently attach more readily, e.g. with greater efficiency, to 5' ends of double stranded polynucleotide target sequences having a 5' terminal triplet nucleotide sequence selected from GGT, GGG, TGT, GTT, TGG, GGA, GTG, CGG, CGT and GCG.

Polynucleotide sequencing applications typically require target polynucleotides to have a particular sequencing adapter attached in order for said target polynucleotides to be sequenced. Maximising the proportion of polynucleotides in a sample which comprise an attached sequencing adapter will in turn maximise the proportion of polynucleotides that are subject to sequencing, and so improve overall sequencing efficiency. Furthermore, by ensuring that target double stranded polynucleotides comprise a 5' terminal triplet nucleotide sequence to which topoisomerase attaches a topoisomerase activated adapter more readily (for example a 5' terminal triplet nucleotide sequence selected from GGT, GGG, TGT, GTT, TGG, GGA, GTG, CGG, CGT and GCG), more consistent adapter attachment can be achieved across multiple different target polynucleotides (for example target amplicons), thus improving subsequent sequencing performance and reducing sampling bias of a library preparation. Accordingly, the method of the disclosure allows increased and/or improved sequencing information to be obtained from a polynucleotide sample.

In a first aspect, the disclosure provides a method of preparing a library of nucleic acids, the method comprising:
(a) providing a plurality of topoisomerase activated adapters, each comprising a Vaccinia virus topoisomerase I bound to a double stranded oligonucleotide;
(b) providing a plurality of double stranded target polynucleotides, each comprising a triplet nucleotide sequence at the 5' end of one or both strands, wherein the triplet nucleotide sequence is selected from GGT, GGG, TGT, GTT, TGG, GGA, GTG, CGG, CGT and GCG; and
(c) contacting the topoisomerase activated adapters with the plurality of double stranded target polynucleotides, such that the topoisomerase covalently links the activated adapters to the 5' ends of the double stranded target polynucleotides, thereby preparing a library of nucleic acids.

The disclosure also provides a method of attaching a nucleic acid adapter to a double stranded target polynucleotide, the method comprising:
(a) providing a topoisomerase activated adapter comprising a Vaccinia virus topoisomerase I bound to a double stranded oligonucleotide;
(b) providing a double stranded target polynucleotide comprising a triplet nucleotide sequence at the 5' end of one or both strands, wherein the triplet nucleotide sequence is selected from GGT, GGG, TGT, GTT, TGG, GGA, GTG, CGG, CGT and GCG; and
(c) contacting the topoisomerase activated adapter with the double stranded target polynucleotide such that the topoisomerase covalently links the activated adapter to the 5' ends of the double stranded target polynucleotide, thereby attaching a nucleic acid adapter to a double stranded target polynucleotide.

The disclosure also provides a method of producing adapted PCR amplicons, the method comprising:
(i) amplifying a first nucleic acid using a first oligonucleotide primer and a second oligonucleotide primer to produce a first amplicon, wherein at least one of the first and second primers comprises a 5' tail which terminates in a triplet nucleotide sequence selected from GGT, GGG, TGT, GTT, TGG, GGA, GTG, CGG, CGT and GCG and amplifying a second nucleic acid using a third primer and a fourth primer to produce a second amplicon, wherein at least one of the third and fourth primers comprises a 5' tail which terminates in a triplet nucleotide sequence that is not GGT, GGG, TGT, GTT, TGG, GGA, GTG, CGG, CGT or GCG; and
(ii) contacting the first amplicon and the second amplicon with vaccinia topoisomerase I activated adapters such that the topoisomerase covalently links an adapter to the 5' end of the first amplicon and to the 5' end of the second amplicon.

The disclosure also provides a kit comprising:
- a vaccinia topoisomerase I
- a double stranded oligonucleotide adapter comprising a topoisomerase target site;
- a primer pair consisting of a first and second oligonucleotide primer,
wherein at least one of the first and second primers is a 5' tailed primer having a triplet nucleotide sequence selected from GGT, GGG, TGT, GTT, TGG, GGA, GTG, CGG, CGT and GCG at the 5' terminus of the tail.

### Brief Description of the Figures

**Figure 1****.** Total read counts obtained following sequencing on Oxford Nanopore Technologies MinION sequencing device of amplicons obtained in Example 1 using control and NNN-tailed primers (all control compared to all NNN-tailed).
**Figure 2****.** Total read counts obtained following sequencing on Oxford Nanopore Technologies MinION sequencing device of amplicons obtained in Example 1 using control and NNN-tailed primers (control compared to NNN-tailed for individual target genes).
**Figure 3****.** Graph of the data presented in Table A, showing total Read Counts obtained with each NNN motif for all six targets.
**Figure 4****.** Percentage template modification for the three different targets for Control and GGK tails. 0x = no adapter attached; 1x = adapter attached to one end of amplicon; 2x = adapters attached to both ends of amplicon; shown left to right for each target.
**Figure 5****.** Read counts obtained with control and GGK-tailed amplicons.
**Figure 6****.** Read counts obtained showing control and GGK-tailed amplicons for three target genes.
**Figure 7****.** Percentage template modification for the 12 different targets for control and GGK tails. 0x = no adapter attached; 1x = adapter attached to one end of amplicon; 2x = adapters attached to both ends of amplicon; shown left to right for each target.
**Figure 8****.** Read count for *E. coli* targets, control amplicons.
**Figure 9****.** Read count for *E. coli* targets, GGK-tailed amplicons.
**Figure 10****.** Percentage template modification for Control, GGK, and ACA tails. 0x = no adapter attached; 1x = adapter attached to one end of amplicon; 2x = adapters attached to both ends of amplicon; shown left to right for each target.

### Detailed Description

The present invention will be described with respect to particular embodiments and with reference to certain drawings but the disclosure is not limited thereto but only by the claims. Any reference signs in the claims shall not be construed as limiting the scope. Of course, it is to be understood that not necessarily all aspects or advantages may be achieved in accordance with any particular embodiment. Thus, for example those skilled in the art will recognize that the disclosed embodiments may be embodied or carried out in a manner that achieves or optimizes one advantage or group of advantages as taught herein without necessarily achieving other aspects or advantages as may be taught or suggested herein.

The disclosure, both as to organization and method of operation, together with features and advantages thereof, may best be understood by reference to the following detailed description when read in conjunction with the accompanying drawings. The aspects and advantages of the invention will be apparent from and elucidated with reference to the embodiment(s) described hereinafter. Reference throughout this specification to "one embodiment" or "an embodiment" means that a particular feature, structure or characteristic described in connection with the embodiment is included in at least one disclosed embodiment. Thus, appearances of the phrases "in one embodiment" or "in an embodiment" in various places throughout this specification are not necessarily all referring to the same embodiment, but may. Similarly, it should be appreciated that in the description of exemplary disclosed embodiments, various features are sometimes grouped together in a single embodiment, figure, or description thereof for the purpose of streamlining the disclosure and aiding in the understanding of one or more of the various inventive aspects. This method of disclosure, however, is not to be interpreted as reflecting an intention that the claimed invention requires more features than are expressly recited in each claim. Rather, as the following claims reflect, inventive aspects lie in less than all features of a single foregoing disclosed embodiment.

It should be appreciated that "embodiments" of the disclosure can be specifically combined together unless the context indicates otherwise. The specific combinations of all disclosed embodiments (unless implied otherwise by the context) are further disclosed embodiments of the claimed invention.

In addition as used in this specification and the appended claims, the singular forms "a", "an", and "the" include plural referents unless the content clearly dictates otherwise. Thus, for example, reference to "a polynucleotide" includes two or more polynucleotides, reference to "a topoisomerase" and the like.

Unless otherwise indicated, nucleic acid sequences herein are written in the 5'-to-3' direction from left to right.

### Method of preparing a library of nucleic acids

The disclosure provides a method of preparing a library of nucleic acids, the method comprising:
(a) providing a topoisomerase activated adapter comprising a Vaccinia virus topoisomerase I bound to a double stranded oligonucleotide or a plurality of topoisomerase activated adapters, each comprising a topoisomerase bound to a double stranded oligonucleotide;
(b) providing a plurality of double stranded target polynucleotides, each comprising a triplet nucleotide sequence at the 5' end of one or both strands, wherein the triplet nucleotide sequence is selected from GGT, GGG, TGT, GTT, TGG, GGA, GTG, CGG, CGT and GCG; and
(c) contacting the topoisomerase activated adapters with the plurality of double stranded target polynucleotides, such that the topoisomerase covalently links the activated adapters to the 5' ends of the double stranded target polynucleotides, thereby preparing a library of nucleic acids.

For example, the method of preparing a library of nucleic acids, the method may comprise:
(a) providing a plurality of topoisomerase activated adapters, each comprising a Vaccinia virus topoisomerase I bound to a double stranded oligonucleotide;
(b) providing a plurality of double stranded target polynucleotides, each comprising a triplet nucleotide sequence at the 5' end of one or both strands, wherein the triplet nucleotide sequence is selected from GGT, GGG, TGT, GTT, TGG, GGA, GTG, CGG and CGT, or from GGT, GGG, TGT, GTT and TGG; and
(c) contacting the topoisomerase activated adapters with the plurality of double stranded target polynucleotides, such that the topoisomerase covalently links the activated adapters to the 5' ends of the double stranded target polynucleotides, thereby preparing a library of nucleic acids.

The library of nucleic acids produced by the method of the disclosure comprises a plurality of double stranded target polynucleotides to which a topoisomerase activated adapter has been added to one or both ends thereof.

The library of nucleic acids may be a sequencing library. The term "sequencing library" may refer to nucleic acids that are prepared for sequencing, for example using Next Generation Sequencing methods, for example using nanopore sequencing methods. The nucleic acids in a sequencing library may optionally be amplified nucleic acids, for example using PCR, for example in the form of amplicons.

### Double stranded target polynucleotides

The double stranded target polynucleotide may comprise a nucleic acid. The nucleic acid may comprise one or more naturally-occurring nucleic acids, such as deoxyribonucleic acid (DNA) and/or ribonucleic acid (RNA). The double stranded target polynucleotides may comprise double stranded DNA or double stranded RNA. The double stranded target polynucleotides may comprise a DNA/RNA duplex, e.g. one strand of RNA hybridized to one strand of DNA.

The nucleic acid may comprise one or more synthetic nucleic acids. Synthetic nucleic acids are known in the art. For example, the nucleic acid may comprises peptide nucleic acid (PNA), glycerol nucleic acid (GNA), threose nucleic acid (TNA), locked nucleic acid (LNA) and/or or other synthetic polymers with nucleotide side chains. If the polynucleotide is PNA, the PNA backbone may be composed of repeating N-(2-aminoethyl)-glycine units linked by peptide bonds. If the polynucleotide is GNA, the GNA backbone may be composed of repeating glycol units linked by phosphodiester bonds. If the polynucleotide is TNA, the TNA backbone may be composed of repeating threose sugars linked together by phosphodiester bonds. If the polynucleotide is LNA, the LNA backbone may be formed from ribonucleotides as discussed above having an extra bridge connecting the 2' oxygen and 4' carbon in the ribose moiety.

The double stranded target polynucleotides may be of any length. For example, the double stranded target polynucleotides may be at least about 10, at least about 50, at least about 70 at least about 100, at least about 150, at least about 200, at least about 250, at least about 300, at least about 400 or at least about 500 nucleotides in length. Double stranded target polynucleotides may be at least about 1,000, at least about 5,000, at least about 10,000, at least about 100,000, at least about 500,000, at least about 1,000,000, or at least about 10,000,000 or more nucleotides in length. The double stranded target polynucleotides is preferably from about 30 to about 10,000, such as from about 50 to about 5,000, from about 100 to about 2,000 nucleotides, or about 500 to about 1,000 nucleotides in length. The double stranded target polynucleotide may itself be a fragment of a longer polynucleotide.

The double stranded target polynucleotides may be linear. The double stranded target polynucleotides may be circular. The target double stranded target polynucleotides may an end-to-end RNA or DNA molecule.

The double stranded target polynucleotide comprises a triplet nucleotide sequence at the 5' end of one or both strands of the target polynucleotide, preferably at the 5' end of both strands of the target polynucleotide, wherein the triplet nucleotide sequence is selected from GGT, GGG, TGT, GTT, TGG, GGA, GTG, CGG, CGT and GCG. In one embodiment, the triplet nucleotide sequence is selected from GKK and KGK, wherein K is G or T. Thus, in one embodiment, the triplet nucleotide sequence is selected from GGG, GTG, GGT, GTT, TGG and TGT. In one embodiment, the triplet nucleotide sequence is GGK. Thus, in one embodiment, the triplet nucleotide sequence is GGG or GGT. Optionally, the triplet nucleotide sequence is GGG. Optionally, the triplet sequence is GGT.

The triplet nucleotide sequence at the 5' end of one or both strands of the plurality of double stranded target polynucleotides may be a first triplet sequence that is immediately 5' to a second triplet nucleotide sequence, i.e. the 3' end of the first triplet sequence is attached to the 5' end of the second triplet sequence, wherein the second triplet nucleotide sequence is selected from GGT, GGG, TGT, GTT, TGG, GGA, GTG, CGG, CGT and GCG. In one embodiment, the second triplet nucleotide sequence is selected from GKK and KGK, wherein K is G or T. Thus, in one embodiment, the second triplet nucleotide sequence is selected from GGG, GTG, GGT, GTT, TGG and TGT. Optionally, the second triplet nucleotide sequence is GGK, i.e. GGT or GGG. Optionally, the second triplet nucleotide sequence is GGG. Optionally, the second triplet nucleotide sequence is GGT. Hence, the double stranded target polynucleotide may comprise a sextuplet nucleotide sequence at the 5' end of one or both strands of the target polynucleotide, preferably at the 5' end of both strands of the target polynucleotide, wherein the sextuplet nucleotide sequence consists of a first triplet nucleotide sequence selected from GGT, GGG, TGT, GTT, TGG, GGA, GTG, CGG, CGT and GCG and a second triplet nucleotide sequence selected from GGT, GGG, TGT, GTT, TGG, GGA, GTG, CGG, CGT and GCG. In one embodiment, the first triplet nucleotide sequence is selected from GKK and KGK, wherein K is G or T. Thus, in one embodiment, the first triplet nucleotide sequence is selected from GGG, GTG, GGT, GTT, TGG and TGT. Optionally, the first triplet nucleotide sequence is GGK, i.e. GGT or GGG. Optionally, the first triplet nucleotide sequence is GGT. Optionally, the first triplet nucleotide sequence is GGG. Accordingly, exemplary sextuplet sequences include GGKGGK, e.g. GGTGGT, GGGGGG, GGTGGG or GGGGGT.

The second triplet sequence may be immediately 5' to a third triplet nucleotide sequence selected from GGT, GGG, TGT, GTT, TGG, GGA, GTG, CGG, CGT and GCG. In one embodiment, the third triplet nucleotide sequence is selected from GKK and KGK, wherein K is G or T. Thus, in one embodiment, the third triplet nucleotide sequence is selected from GGG, GTG, GGT, GTT, TGG and TGT. Optionally, the third triplet nucleotide sequence is GGK, i.e. GGT or GGG. Optionally, the third triplet nucleotide sequence is GGG. Optionally, the third triplet nucleotide sequence is GGT. Hence, the double stranded target polynucleotide may comprise a 9mer nucleotide sequence at the 5' end of one or both strands of the target polynucleotide, preferably at the 5' end of both strands of the target polynucleotide, wherein the 9mer nucleotide sequence consists of a first triplet nucleotide sequence selected from GGT, GGG, TGT, GTT, TGG, GGA, GTG, CGG, CGT and GCG, a second triplet nucleotide sequence selected from GGT, GGG, TGT, GTT, TGG, GGA, GTG, CGG, CGT and GCG, and a third triplet nucleotide sequence selected from GGT, GGG, TGT, GTT, TGG, GGA, GTG, CGG, CGT and GCG. Exemplary 9mer sequences include GGKGGKGGK, e.g. GGTGGTGGT, GGTGGTGGG, GGGGGGGGG, GGGGGGGGT, GGTGGGGGT, GGTGGGGGG, GGGGGTGGT or GGGGGTGGG.

The double stranded target polynucleotides may be blunt-ended. The double stranded target polynucleotides may be produced using a blunting polymerase. Blunting polymerases, such as Q5 from NEB, are well known in the art.

The double stranded target polynucleotides may comprise a nucleotide overhang, optionally at the 3' overhang. The overhang may be generated by polymerases known in the art. The 3' overhang may, for example, be an 'A-tail' comprising a single dA nucleotide. The A-tailed overhang may be produced using any known method in the art.

The ending of the double stranded target polynucleotide is preferably complementary to the ending of the adapter comprised within the topoisomerase-activated adapter as described herein, e.g. a blunt ended adapter may typically be used together with a blunt ended target polynucleotide and a dT-tailed adapter may typically be used with a dA-tailed double stranded target polynucleotide.

### Plurality of double stranded target polynucleotides

The method of preparing a library of nucleic acids involves providing a plurality of double stranded target polynucleotides. Accordingly, at least two double stranded target polynucleotides are provided in the method. For example, at least about 5, at least about 10, at least about 50 or at least about 100 double stranded target polynucleotides may be provided.

The plurality of double stranded target polynucleotides may be present in a sample. The invention is typically carried out on a sample that is known to contain or suspected to contain a plurality of double stranded target polynucleotides.

The sample may be a biological sample. The methods may be carried out in vitro using a sample obtained from or extracted from any organism or microorganism. The organism or microorganism is typically archaeal, prokaryotic or eukaryotic and typically belongs to one of the five kingdoms: plantae, animalia, fungi, monera and protista. The invention may be carried out in vitro on a sample obtained from or extracted from any virus. The sample is preferably a fluid sample. The sample typically comprises a body fluid of the patient. The sample may be urine, lymph, saliva, mucus or amniotic fluid but is preferably blood, plasma or serum.

Typically, the sample is human in origin, but alternatively it may be from another mammal animal such as from commercially farmed animals such as horses, cattle, sheep, fish, chickens or pigs or may alternatively be pets such as cats or dogs. Alternatively, the sample may be of plant origin, such as a sample obtained from a commercial crop, such as a cereal, legume, fruit or vegetable, for example wheat, barley, oats, canola, maize, soya, rice, rhubarb, bananas, apples, tomatoes, potatoes, grapes, tobacco, beans, lentils, sugar cane, cocoa, cotton.

The sample may be a non-biological sample. The non-biological sample is preferably a fluid sample. Examples of non-biological samples include surgical fluids, water such as drinking water, sea water or river water, and reagents for laboratory tests. The sample is typically processed prior to being used in the invention, for example by centrifugation or by passage through a membrane that filters out unwanted molecules or cells, such as red blood cells. The sample may be subject to the method disclosed herein immediately upon being taken. The sample may also be typically stored prior to assay, preferably below -70°C.

Each of the double stranded target polynucleotides may comprise a triplet nucleotide sequence at the 5' end of one or both strands of the target polynucleotide, preferably at the 5' end of both strands of the target polynucleotide, wherein the triplet nucleotide sequence is selected from GGT, GGG, TGT, GTT, TGG, GGA, GTG, CGG, CGT and GCG as described above, such as from GGT, GGG, TGT, GTT, TGG, GGA, GTG, CGG and CGT or from GGT, GGG, TGT, GTT and TGG.

In some embodiments, the plurality of double stranded target polynucleotides may comprise one or more, such as two, five, ten or more, double stranded polynucleotide comprising a triplet nucleotide sequence at the 5' end of one or both strands of the target polynucleotide, preferably at the 5' end of both strands of the target polynucleotide, wherein the triplet nucleotide sequence is selected from CAA, ATG, AAT, TAC, GCG, TAG, AAC, ATC, GCA, GCC, TAA, TTT, ATA, AAG, CCT, AAA, CCG, TTG, TTC, TCG, TCA, CCC, CCA, TCT, ACT, ACC, TCC, ACG, TTA and ACA, such as from ACA, TTA, ACG, TCC, ACC, ACT, TCT, CCA, CCC, TCA, TCG, TTC, TTG, CCG and AAA; optionally wherein the triplet sequence is ACA.

### Producing the double stranded polynucleotide

The method may further comprise producing the double stranded target polynucleotide, such as the plurality of double stranded polynucleotides, comprising the triplet nucleotide sequence at the 5' end of one or both strands by PCR (polymerase chain reaction) using a first oligonucleotide primer and a second oligonucleotide primer, wherein at least one of the first and second primers comprises a 5' tail which terminates in the triplet nucleotide sequence.

The plurality of double stranded target polynucleotides comprising a triplet nucleotide sequence at the 5' end of both strands may be produced through the use of first and second oligonucleotide primers each comprising a 5' tail which terminates in the triplet nucleotide sequence.

Oligonucleotide primers having a 5' tail are known in the art as 'tailed' primers. Tailed primers comprise a 5' sequence of nucleotides (a 'tail') that is non-complementary to a nucleotide sequence in a given polynucleotide target, together with a 3' sequence of nucleotides (the primer sequence) that is complementary to a specific sequence in the given polynucleotide target. Thus, the 5' tail of the primer may be non-complementary to a nucleotide sequence present at the primer-binding site in a target polynucleotide. In aspects of the present disclosure which comprise producing the plurality of double stranded target polynucleotides comprising the triplet nucleotide sequence at the 5' end of one or both strands, the 5' tail terminates in the triplet nucleotide sequence selected from GGT, GGG, TGT, GTT, TGG, GGA, GTG, CGG, CGT and GCG, such as from GGT, GGG, TGT, GTT, TGG, GGA, GTG, CGG and CGT or from GGT, GGG, TGT, GTT and TGG; thus, the triplet nucleotide sequence is present at the 5' end of the 5' tail. In some embodiments, the 5' tail terminates in GKK or KGK, wherein K is G or T. Thus, in some embodiments, the 5' tail terminates in GGG, GTG, GGT, GTT, TGG or TGT. In some embodiments, the 5' tail terminates in GGK. Thus, in some embodiments, the 5' tail terminates in GGG or GGT. In some embodiments, the 5' tail terminates in GGG. In some embodiments, the 5' tail terminates in GGT. The result of using such tailed primers is that the 5' tail triplet sequence is incorporated into the 5' ends of the amplicons which are generated from the step of PCR. Such amplicons constitute double stranded target polynucleotides. Advantageously, the use of 5' tailed primers as described herein allows the triplet nucleotide sequences of the invention to be appended to the 5' end of known primer sequences for a given target nucleic acid sequence that it is desired to amplify, thus providing the advantages of the present invention when employing such known primer sequences in PCR.

The 5' tail may consist of the triplet nucleotide sequence, or comprise the triplet nucleotide sequence at the 5' end. The 5' tail may be of any suitable length, for example from 3 to about 15 nucleotides, such as from about 4, 5 or 6 nucleotides to about 8, 9, 10 or 12 nucleotides. In some embodiments, the triplet nucleotide sequence at the 5' end of the tail is followed by up to about 3, about 6, about 9, or about 12 additional N nucleotides, wherein N is A, C, G or T; for example 1, 2, 3, 4, 5, 6, 7, 8, 9, 10, 11 or 12 additional N nucleotides; for example, NNN, NNNNNN, NNNNNNNNN, or NNNNNNNNNNNN. Thus, in some embodiments, the 5' tail may comprise (from the 5' end) a first triplet nucleotide sequence selected from GGT, GGG, TGT, GTT, TGG, GGA, GTG, CGG, CGT and GCG; or selected from GKK and KGK; or of GGK; followed by the nucleotide sequence NNN, or NNNNNN, or NNNNNNNNN, or NNNNNNNNNNNN, wherein N is A, C, G or T.

The 5' tail may comprise or consist of a sextuplet nucleotide sequence, wherein the sextuplet nucleotide sequence consists of a first triplet nucleotide sequence selected from GGT, GGG, TGT, GTT, TGG, GGA, GTG, CGG, CGT and GCG and a second triplet nucleotide sequence selected from GGT, GGG, TGT, GTT, TGG, GGA, GTG, CGG, CGT and GCG. The first and second triplet sequences may, for example, each be independently selected from GGT, GGG, TGT, GTT, TGG, GGA, GTG, CGG and CGT or from GGT, GGG, TGT, GTT and TGG. In one embodiment, the first triplet nucleotide sequence is selected from GKK and KGK, wherein K is G or T. Thus, in one embodiment, the first triplet nucleotide sequence is selected from GGG, GTG, GGT, GTT, TGG and TGT. In one embodiment, the first triplet nucleotide sequence is GGK, i.e. GGT or GGG. Optionally, the first triplet nucleotide sequence is GGT. Accordingly, exemplary sextuplet sequences include GGKKKK and GGKGGK, e.g. GGTGGT, GGGGGG, GGTGGG or GGGGGT.

The 5' tail may comprise or consist of a 9mer nucleotide sequence, wherein the 9mer nucleotide sequence consists of a first triplet nucleotide sequence selected from GGT, GGG, TGT, GTT, TGG, GGA, GTG, CGG, CGT and GCG, a second triplet nucleotide sequence selected from GGT, GGG, TGT, GTT, TGG, GGA, GTG, CGG, CGT and GCG and a third triplet nucleotide sequence selected from GGT, GGG, TGT, GTT, TGG, GGA, GTG, CGG, CGT and GCG. The first, second and third triplet sequences may, for example, each be independently selected from GGT, GGG, TGT, GTT, TGG, GGA, GTG, CGG and CGT or from GGT, GGG, TGT, GTT and TGG. Exemplary 9mer sequences include GGKKKKKKK and GGKGGKKKK, GGKGGKGGK, e.g. GGTGGTGGT, GGTGGTGGG, GGGGGGGGG, GGGGGGGGT, GGTGGGGGT, GGTGGGGGG, GGGGGTGGT or GGGGGTGGG. In aspects of the disclosure comprising the production of the plurality of double stranded target polynucleotides comprising the triplet nucleotide sequence at the 5' end of one or both strands by PCR, the PCR step may be performed using any suitable polymerase known in the art. In particular, the suitable polymerase may be determined by the desire of the user to generate double stranded target polynucleotide amplicons with blunt ends or with nucleotide overhang ends. Such polymerases are well known in the art.

### Topoisomerase activated adapters

Topisomerases, such as Vaccinia virus topoisomerase I, act *in vivo* to help regulate positive and negative supercoiling in DNA. Vaccinia virus topoisomerase I can bind to duplex DNA and cleave the phosphodiester backbone of one strand at the 3' end of the target sequence (C/T)CCTT. In the cleavage reaction, bond energy is conserved via the formation of a covalent adduct between the 3' phosphate of the incised strand and Tyr-274 of the topoisomerase protein. Topoisomerase can re-ligate the covalently held strand across the same bond originally cleaved (as occurs during relaxation of supercoiled DNA), or it can re-ligate to a heterologous acceptor DNA and thereby create a recombinant molecule. Dissociation of the nucleic acid associated with the free 5' end created by the topoisomerase cleavage event enables another nucleic acid fragment with a compatible end including a free 5'-OH to be joined to the activated topoisomerase-DNA complex. Vaccinia DNA topoisomerase is described in Cheng & Shuman (Nucleic Acids Research, 2000, Vol. 28, No. 9, 1893-1898).

The methods described herein may comprise providing a topoisomerase activated adapter, comprising a Vaccinia virus topoisomerase I bound to a double stranded oligonucleotide. Thus, the adapters may be topoisomerase activated polynucleotide adapters. The term "topoisomerase activated adapter" may refer to a polynucleotide structure comprising a duplex oligonucleotide region having a topoisomerase covalently bound at or near the 3' terminus of a first end. In one embodiment, the topoisomerase activated adapter is not a cloning vector.

The double stranded oligonucleotide may comprise a nucleic acid. The nucleic acid may comprise one or more naturally-occurring nucleic acids, such as deoxyribonucleic acid (DNA) and/or ribonucleic acid (RNA). The double stranded target polynucleotides may comprise double stranded DNA or double stranded RNA. The double stranded target polynucleotides may comprise a DNA/RNA duplex, e.g. one strand of RNA hybridized to one strand of DNA.

The nucleic acid may comprise one or more synthetic nucleic acids. Synthetic nucleic acids are known in the art. For example, the nucleic acid may comprises peptide nucleic acid (PNA), glycerol nucleic acid (GNA), threose nucleic acid (TNA), locked nucleic acid (LNA) and/or or other synthetic polymers with nucleotide side chains. If the polynucleotide is PNA, the PNA backbone may be composed of repeating N-(2-aminoethyl)-glycine units linked by peptide bonds. If the polynucleotide is GNA, the GNA backbone may be composed of repeating glycol units linked by phosphodiester bonds. If the polynucleotide is TNA, the TNA backbone may be composed of repeating threose sugars linked together by phosphodiester bonds. If the polynucleotide is LNA, the LNA backbone may be formed from ribonucleotides as discussed above having an extra bridge connecting the 2' oxygen and 4' carbon in the ribose moiety.

The double stranded oligonucleotides may be of any suitable length, such as from about 10 to about 100 nucleotides, such as about 40, 50, 60, 70, 80 or 90 nucleotides. The double stranded nucleotide may comprise a single stranded overhang at one or both ends. The single stranded overhang may be of any suitable length, such as from 1 to about 30 nucleotides, for example at least about 5, about 10, about 15 or about 20 nucleotides.

The adapter typically comprises a topoisomerase target site. Preferably, the topoisomerase target site comprises a [C/T]CCTT nucleotide sequence, for example CCCTT or TCCTT. Topoisomerase is known to recognise [C/T]CCTT sites in double stranded DNA, cleave the backbone of double stranded DNA and become covalently bound to the 3' hydroxyl group of the cleaved backbone, thereby generating a topoisomerase activated adapter and a leaving group. The topoisomerase may then attach the adapter to the 5' end of a double stranded target polynucleotide. The adapter covalently attached to the topoisomerase may preferably be blunt-ended or have a nucleotide overhang, such as a 5' dT-tail. The types of ending to a topoisomerase adapter are well known in the art and may be selected on the basis of the corresponding end of a double stranded target polynucleotide, such that the ends of the adapter and double stranded target polynucleotide are complementary. For example, if the double stranded target polynucleotide is blunt-ended, the adapter is preferably also blunt ended or if the double stranded target polynucleotide is dA-tailed, the adapter is preferably dT-tailed.

Preferably, the adapter comprises a [C/T]CCTT sequence at its 3' terminus. Such a sequence allows the generation of a topoisomerase activated adapter by virtue of the adapter first being comprised within a double stranded DNA sequence, thereby allowing the topoisomerase to cleave the DNA backbone, covalently bind to the 3' hydroxyl group of the [C/T]CCTT sequence, thereby generating a topoisomerase activated adapter.

The adapter may further comprise any component required to further functionalise the adapter. Such components are known in the art. The adapter may comprise a click reactive group, a barcode, a fluorophore, a conjugation agent, a pull down group, a tethering moiety, a marker, a modified base, an abasic residue, a sequencing adapter, an intermediate adapter, an amplification adapter, a hairpin adapter, a unique molecular identifier, a helicase binding site and/or a spacer. Examples of polynucleotide sequencing adapters suitable for use in nanopore sequencing are described in WO 2015/110813 and WO 2020/234612, The adapter may comprise a component such as described above, for example a click group, that enables the subsequent attachment to the adapter of a further adapter such as a polynucleotide sequencing adapter. Thus, by way of example, the attachment of a topoisomerase activated adapter to a target double stranded polynucleotide, such as described herein, may facilitate the subsequent attachment of a polynucleotide sequencing adapter.

The adapter may comprise a marker. The marker may be any suitable marker that enables the skilled person to identify where or whether the one or more adapters have been attached to the double stranded target polynucleotide. Exemplary markers include one or more biotin molecules; one or more modified bases; one or more abasic residues; one or more base-base conjugates; or one or more protein-base conjugates.

Preferably, the marker is detectable by one or more sequencing technologies known in the art, such as by nanopore-based sequencing. The marker may be optically detectable. For example, the marker may fluoresce under excitation by the appropriate wavelength of light. For example, the marker may comprise one or more fluorescent bases, for example Cy3 or Cy5. Any optical and/or fluorescent marker deemed suitable by the skilled person may be used. Other detectable markers include non-canonical bases, abasics and spacers. Any abasic and/or spacer deemed suitable by the skilled person may be used. Particularly, exemplary spacers may include C3, PC Spacer, Hexanediol, Spacer 9, Spacer 18 or 1',2'-Dideoxyribose (dSpacer).

The adapter may comprise one or more modified bases. The modified base can be any suitable modified base. The modified base may be, for example, a nucleotide labelled with biotin (i.e. a biotinylated nucleotide), or a nucleotide labelled with digoxigenin (i.e. a digoxigenin-labelled nucleotide). Modified bases such as biotin-labelled or digoxigenin-labelled bases may enable coupling of the double stranded target polynucleotide to a solid surface, for example a surface coated with streptavidin or anti-digoxigenin respectively.

The adapter may comprise a pull-down group. The pull-down group may be any suitable pull-down group that enables the skilled person to purify or isolate the double stranded target polynucleotide or immobilize the double stranded target polynucleotide by attaching the double stranded target polynucleotide to another substance. The other substance may, for example, be a nucleic acid construct, a nucleic acid molecule, a polypeptide, a protein, a membrane or a solid phase surface. Exemplary pull-down groups include one or more polypeptides, and one or more hydrophobic anchors.

The pull-down group may, for example, comprise one or more modified nucleotide bases. The modified base may be a nucleotide labelled with biotin (i.e. a biotinylated nucleotide), or a nucleotides labelled with digoxigenin (i.e. a digoxigenin-labelled nucleotide). Modified bases within the pull-down group such as biotin- or digoxigenin-labelled bases may enable tethering of the double stranded target polynucleotide to a solid surface, for example, a surface coated with streptavidin or anti-digoxigenin. Any suitable tether that enables coupling to a solid surface may be used. Solid surfaces that could be coupled to double stranded target polynucleotides prepared by the methods of the disclosure described herein may, for example, include nanogold, polystyrene bead and Qdot.

The pull-down group may, for example, comprise a tethering moiety wherein the tethering moiety comprises a hydrophobic anchor, optionally wherein the hydrophobic anchor comprises a hydrophobic nucleotide base. The tethering moiety may be a lipid, fatty acid, sterol, carbon nanotube, protein or amino acid, cholesterol, palmitate or octyl tocopherol.

The adapter may permit further manipulation of the double stranded target polynucleotide or may permit direct sequencing of the double stranded target polynucleotide (e.g. a sequencing adapter). The adapter may be any adapter deemed suitable by the skilled person.

Adapters are known in the art. The adapter may, for example, comprise a nucleotide sequence enabling protein binding, a sequencing adapter, a PCR adapter, a hairpin adapter, an adapter that would enable circularization of a target polynucleotide and/or rolling circle amplification, a unique molecular identifier (UMI), an oligonucleotide splint, a click chemistry moiety, a exonuclease-resistant bases and/or phosphorothioate bonds. The adapter may concurrently be bound by a desired protein, for example, a motor enzyme.

The adapter may comprise RNA and/or DNA sequences that can be recognized and bound by a DNA and/or RNA binding protein. For example, in the method described herein, the adapter may be bound by a motor enzyme such as a helicase or a translocase.

The adapter may be a sequence motif that may be specifically recognized by a particular DNA and/or RNA binding protein. The adapter may be an RNA/DNA hybrid sequence that may be specifically recognized and bound by a DNA and/or RNA binding protein. For instance, sequence motifs may be recognized by DNA-binding proteins characterised by their structural domains e.g. helix-turn-helix, zinc finger, leucine zipper, winged helix, winged helix-turn-helix, helix-loop-helix, HMG-box, Wor3 and OB-fold. The adapter may be a lacO or tetO array capable of being bound by lac or tet repressor proteins. The adapter may be an RNA-DNA hybrid sequence capable of being bound by an antibody. The RNA-DNA hybrid marker may be bound by a S9.6 antibody.

The adapter may comprise a nucleotide sequence suitable for oligonucleotide hybridisation. Particularly, oligonucleotides may comprise complementary bases for hybridizing to the adapter, thus enabling priming of the extension (linear amplification) of a complementary polynucleotide sequence or polymerase chain reaction

The adapter may comprise a nucleotide sequence that may act as a unique molecular identifier (UMI). The UMI may be detectable by any sequencing method deemed suitable by the skilled person. Particularly, the UMI may be used to detect and quantify the double stranded target polynucleotide. Target double stranded polynucleotide sequencing reads may be clustered by the presence of a UMI, thus enabling the improvement of single molecule sequencing accuracy.

The adapter may be any adapter amenable to form a covalent bond to other molecules, for example via click chemistry. The adapter may comprise a group that allows copper-free click chemistry. An exemplary group applicable to the adapters of the present method is a 5'DBCO group.

### Method of determining the presence or absence or one or more characteristics of the double stranded target polynucleotides covalently linked to the activated adapters

The products of any of the methods described herein, such as the double stranded target polynucleotide with a covalently attached adapter, or the double stranded polynucleotides of the nucleic acid library comprising double stranded target polynucleotides, may be subject to a nanopore based method to determine the presence, absence or one or more characteristics of said products. The methods of the disclosure may therefore further comprise determining the presence, absence or one or more characteristics of a target double stranded target polynucleotide covalently linked to an adapter by a topoisomerase as described above. The presence, absence or one or more characteristics of such a target double stranded target polynucleotide may be determined by:
(a) contacting the target polynucleotide with a nanopore or a mutant thereof such that the target polynucleotide moves with respect to the pore; and
(b) taking one or more measurements as the polynucleotide moves with respect to the pore,
thereby determining the presence, absence or one or more characteristics of the polynucleotide.

Nanopore-based methods of detecting a target polynucleotide in a sample have been described previously (WO 2018/060740). Nanopore-based methods for characterising a target polynucleotide in a sample have been described previously (WO 2015/124935). Accordingly, any suitable nanopore-based method of polynucleotide characterisation would be suitable for application to the method of determining the presence absence or one or more characteristics of the double stranded target polynucleotides covalently linked to the activated adapters described herein.

The method may further comprise monitoring for the presence or absence of an effect on the potential difference being applied across the membrane as a result of the interaction of the target polynucleotide with the transmembrane pore, thereby determining the presence or absence of the target polynucleotide. The effect is indicative of the double stranded target polynucleotide interacting with the transmembrane pore. The effect may be caused by the translocation of one or both strands of the double stranded target polynucleotide through the pore, of an adapter attached to one of the components of the double stranded target polynucleotide. The effect may be monitored using an electrical measurement and/or an optical measurement. In this case, the effect is a measured change or measured changes in an electrical or optical quantity. The electrical measurement may be a current measurement, an impedance measurement, a tunnelling measurement or a field effect transistor (FET) measurement. The effect may be a change in ion flow through the transmembrane pore resulting in a change in current, resistance or a change in an optical property. The effect may be electron tunneling across the transmembrane pore. The effect may be a change in potential due to the interaction of the double stranded target polynucleotide with the transmembrane pore wherein the effect is monitored using localized potential sensor in a FET measurement.

In the method of characterising a double stranded target polynucleotide described herein, the contacting of the double stranded target polynucleotide with the pore is such that at least one nucleic acid strand of the double stranded target polynucleotide moves through the pore.

In the method of characterising a double stranded target polynucleotide described herein, the taking of one or more measurements are indicative of one or more characteristics of the double stranded target polynucleotide are selected from (i) the length of the polynucleotide, (ii) the identity of the polynucleotide, (iii) the sequence of the polynucleotide, (iv) the secondary structure of the polynucleotide and (v) whether or not the polynucleotide is modified.

Step (b) comprises contacting the target polynucleotide with a transmembrane pore such that the modified polynucleotide moves through the pore. The target polynucleotide may be contacted with a transmembrane pore such they both move through the pore.

The method is preferably carried out with a potential applied across the pore. The applied potential may be a voltage potential. Alternatively, the applied potential may be a chemical potential. An example of this is using a salt gradient across an amphiphilic layer. A salt gradient is disclosed in Holden et al., J Am Chem Soc. 2007 Jul 11; 129(27):8650-5. In some instances, the current passing through the pore as the polynucleotide moves with respect to the pore is used to determine the sequence of the double stranded target polynucleotide. This is strand sequencing.

The whole or only part of the double stranded target polynucleotide may be characterised, for instance sequenced, using this method of characterising a target polynucleotide.

A transmembrane pore is a structure that crosses the membrane to some degree. It permits hydrated ions driven by an applied potential to flow across or within the membrane. The transmembrane pore typically crosses the entire membrane so that hydrated ions may flow from one side of the membrane to the other side of the membrane. However, the transmembrane pore does not have to cross the membrane. It may be closed at one end. For instance, the pore may be a well, gap, channel, trench or slit in the membrane along which or into which hydrated ions may flow.

Any suitable transmembrane pore may be used. The pore may be biological or artificial. Suitable pores include, but are not limited to, protein pores, polynucleotide pores and solid state pores. In any of the methods described herein, the pore may permit translocation of double-stranded polynucleotide and bound polynucleotide through the pore. In any of the methods described herein, the pore may permit translocation of double-stranded polynucleotide and bound polynucleotide through the pore. In any of the methods described herein, the pore may permit translocation of a double-stranded polynucleotide. In any of the methods described herein, the pore may permit translocation of a single-stranded polynucleotide.

The pore may be present in any suitable membrane. Suitable membranes are well-known in the art. The membrane is preferably an amphiphilic layer. An amphiphilic layer is a layer formed from amphiphilic molecules, such as phospholipids, which have both at least one hydrophilic portion and at least one lipophilic or hydrophobic portion. The amphiphilic layer may be a monolayer or a bilayer. The amphiphilic molecules may be synthetic or naturally occurring. Non-naturally occurring amphiphiles and amphiphiles which form a monolayer are known in the art and include, for example, block copolymers (Gonzalez-Perez et al., Langmuir, 2009, 25, 10447-10450). Block copolymers are polymeric materials in which two or more monomer sub-units are polymerized together to create a single polymer chain. Block copolymers typically have properties that are contributed by each monomer sub-unit. However, a block copolymer may have unique properties that polymers formed from the individual sub-units do not possess. Block copolymers can be engineered such that one of the monomer sub-units is hydrophobic (i.e. lipophilic), whilst the other sub-unit(s) are hydrophilic whilst in aqueous media. In this case, the block copolymer may possess amphiphilic properties and may form a structure that mimics a biological membrane. The block copolymer may be a diblock (consisting of two monomer sub-units), but may also be constructed from more than two monomer sub-units to form more complex arrangements that behave as amphipiles. The copolymer may be a triblock, tetrablock or pentablock copolymer.

The amphiphilic layer may be a planar lipid bilayer or a supported bilayer.

The amphiphilic layer may be a lipid bilayer. Lipid bilayers are models of cell membranes and serve as excellent platforms for a range of experimental studies. For example, lipid bilayers can be used for in vitro investigation of membrane proteins by single-channel recording. Alternatively, lipid bilayers can be used as biosensors to detect the presence of a range of substances. The lipid bilayer may be any lipid bilayer. Suitable lipid bilayers include, but are not limited to, a planar lipid bilayer, a supported bilayer or a liposome. The lipid bilayer may be a planar lipid bilayer. Suitable lipid bilayers are disclosed in International Application No. PCT/GB08/000563 (published as WO 2008/102121), International Application No. PCT/GB08/004127 (published as WO 2009/077734) and International Application No. PCT/GB2006/001057 (published as WO 2006/100484).

Methods for forming lipid bilayers are known in the art. Suitable methods are disclosed in the Example. Lipid bilayers are commonly formed by the method of Montal and Mueller (Proc. Natl. Acad. Sci. USA., 1972; 69: 3561-3566). Other common methods of bilayer formation include tip-dipping, painting bilayers and patch-clamping of liposome bilayers.

The lipid bilayer may be formed as described in International Application No. PCT/GB08/004127 (published as WO 2009/077734).

The membrane may be a solid state layer. A solid-state layer is not of biological origin. In other words, a solid state layer is not derived from or isolated from a biological environment such as an organism or cell, or a synthetically manufactured version of a biologically available structure. Solid state layers can be formed from both organic and inorganic materials including, but not limited to, microelectronic materials, insulating materials such as Si₃N₄, Al₂O₃, and SiO, organic and inorganic polymers such as polyamide, plastics such as Teflon^{®} or elastomers such as two-component addition-cure silicone rubber, and glasses. The solid state layer may be formed from monatomic layers, such as graphene, or layers that are only a few atoms thick. Suitable graphene layers are disclosed in International Application No. PCT/US2008/010637 (published as WO 2009/035647).

The method is typically carried out using (i) an artificial amphiphilic layer comprising a pore, (ii) an isolated, naturally-occurring lipid bilayer comprising a pore, or (iii) a cell having a pore inserted therein. The method is typically carried out using an artificial amphiphilic layer, such as an artificial lipid bilayer. The layer may comprise other transmembrane and/or intramembrane proteins as well as other molecules in addition to the pore. Suitable apparatus and conditions are discussed below. The method of the disclosure is carried out in vitro.

The double stranded target polynucleotide may be coupled to the membrane. This may be done using any known method. Particularly, the double stranded target polynucleotide may be coupled to the membrane via a suitable modified polynucleotide ligated to the fragmented target polynucleotide. Alternatively, the modified polynucleotide ligated to the fragmented target polynucleotide may be modified in order to introduce a coupling or anchor element for coupling the double stranded target polynucleotide to a membrane. If the membrane is an amphiphilic layer, such as a lipid bilayer (as discussed in detail above), the double stranded target polynucleotide is preferably coupled to the membrane via a polypeptide present in the membrane or a hydrophobic anchor present in the membrane. The hydrophobic anchor is preferably a lipid, fatty acid, sterol, carbon nanotube or amino acid.

The double stranded target polynucleotide may be coupled directly to the membrane. The polynucleotide is preferably coupled to the membrane via a linker. Preferred linkers include, but are not limited to, polymers, such as polynucleotides, polyethylene glycols (PEGs) and polypeptides. If a polynucleotide is coupled directly to the membrane, then some data will be lost as the characterising run cannot continue to the end of the double stranded target polynucleotide due to the distance between the membrane and the pore. If a linker is used, then the polynucleotide can be processed to completion. If a linker is used, the linker may be attached to the polynucleotide at any position. The linker is preferably attached to the double stranded target polynucleotide at the tail polymer.

The coupling may be stable or transient. For certain applications, the transient nature of the coupling is preferred. If a stable coupling molecule were attached directly to either the 5' or 3' end of a polynucleotide, then some data will be lost as the characterising run cannot continue to the end of the polynucleotide due to the distance between the bilayer and the pore. If the coupling is transient, then when the coupled end randomly becomes free of the bilayer, then the polynucleotide can be processed to completion. Chemical groups that form stable or transient links with the membrane are discussed in more detail below. The polynucleotide may be transiently coupled to an amphiphilic layer, such as a lipid bilayer using cholesterol or a fatty acyl chain. Any fatty acyl chain having a length of from 6 to 30 carbon atoms, such as hexadecanoic acid, may be used.

Suitable methods of coupling are disclosed in WO 2012/164270, WO 2015/110813 and WO 2015/150787.

A common technique for the amplification of sections of genomic DNA is using polymerase chain reaction (PCR). Here, using two synthetic oligonucleotide primers, a number of copies of the same section of DNA can be generated, where for each copy the 5' of each strand in the duplex will be a synthetic polynucleotide. By using an antisense primer that has a reactive group, such as a cholesterol, thiol, biotin or lipid, each copy of the amplified target DNA will contain a reactive group for coupling.

The transmembrane pore is preferably a transmembrane protein pore. A transmembrane protein pore is a polypeptide or a collection of polypeptides that permits hydrated ions, such as analyte, to flow from one side of a membrane to the other side of the membrane. In the present disclosure, the transmembrane protein pore is capable of forming a pore that permits hydrated ions driven by an applied potential to flow from one side of the membrane to the other. The transmembrane protein pore preferably permits analyte such as nucleotides to flow from one side of the membrane, such as a lipid bilayer, to the other. The transmembrane protein pore allows a polynucleotide, such as DNA or RNA, to be moved through the pore.

The transmembrane protein pore may be a monomer or an oligomer. The pore is may be made up of several repeating subunits, such as 6, 7, 8 or 9 subunits. The pore may be a hexameric, heptameric, octameric or nonameric pore.

The transmembrane protein pore typically comprises a barrel or channel through which the ions may flow. The subunits of the pore typically surround a central axis and contribute strands to a transmembrane β barrel or channel or a transmembrane α-helix bundle or channel.

The barrel or channel of the transmembrane protein pore typically comprises amino acids that facilitate interaction with analytes, such as nucleotides, polynucleotides or nucleic acids. These amino acids are preferably located near a constriction of the barrel or channel. The transmembrane protein pore typically comprises one or more positively charged amino acids, such as arginine, lysine or histidine, or aromatic amino acids, such as tyrosine or tryptophan. These amino acids typically facilitate the interaction between the pore and nucleotides, polynucleotides or nucleic acids.

### Method of attaching a polynucleotide adapter to a double stranded target polynucleotide

The disclosure provides a method of attaching a polynucleotide adapter to a double stranded target polynucleotide, the method comprising:
(a) providing a topoisomerase activated adapter comprising a Vaccinia virus topoisomerase I bound to a double stranded oligonucleotide;
(b) providing a double stranded target polynucleotide comprising a triplet nucleotide sequence at the 5' end of one or both strands, wherein the triplet nucleotide sequence is selected from GGT, GGG, TGT, GTT, TGG, GGA, GTG, CGG, CGT and GCG, for example from GGT, GGG, TGT, GTT, TGG, GGA, GTG, CGG and CGT or from GGT, GGG, TGT, GTT and TGG; and
(c) contacting the topoisomerase activated adapter with the double stranded target polynucleotide such that the topoisomerase covalently links the activated adapter to the 5' ends of the double stranded target polynucleotide,
thereby attaching a polynucleotide adapter to a double stranded target.

The features of this method are described in detail above with respect to the method of preparing a nucleic acid library. Any of the features described in respect of the method of preparing a nucleic acid library may equally apply to the method of attaching a polynucleotide adapter to a double stranded target polynucleotide.

The requirements for attaching an adapter to a double stranded target polynucleotide using a topoisomerase activated adapter are well known in the art. Accordingly any suitable reaction conditions may be used, for example those described in the Examples herein.

### Method of producing adapted PCR amplicons

The disclosure provides a method of producing adapted PCR amplicons, the method comprising:
(i) amplifying a first nucleic acid using a first oligonucleotide primer and a second oligonucleotide primer to produce a first amplicon, wherein at least one of the first and second primers comprises a 5' tail which terminates in a triplet nucleotide sequence selected from GGT, GGG, TGT, GTT, TGG, GGA, GTG, CGG, CGT and GCG, such as a triplet nucleotide sequence selected from GGT, GGG, TGT, GTT, TGG, GGA, GTG, CGG and CGT or from GGT, GGG, TGT, GTT and TGG, and amplifying a second nucleic acid using a third primer and a fourth primer to produce a second amplicon, wherein at least one of the third and fourth primers comprises a 5' tail which terminates in a triplet nucleotide sequence that is not GGT, GGG, TGT, GTT, TGG, GGA, GTG, CGG, CGT or GCG, such as a triplet nucleotide sequence selected from GGT, GGG, TGT, GTT, TGG, GGA, GTG, CGG and CGT or from GGT, GGG, TGT, GTT and TGG; and
(ii) contacting the first amplicon and the second amplicon with topoisomerase activated adapters such that the Vaccinia virus topoisomerase I covalently links an adapter to the 5' end of the first amplicon and to the 5' end of the second amplicon.

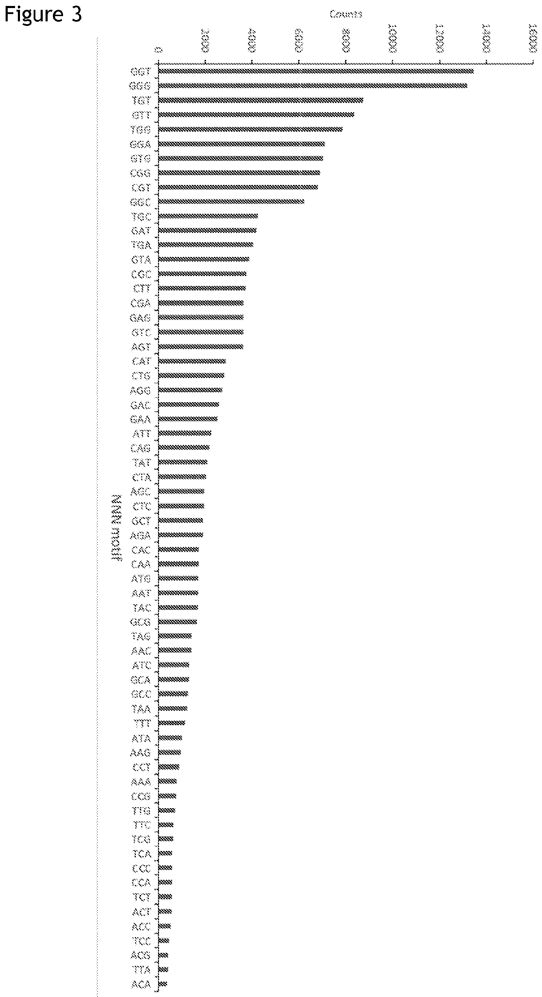

As described above, the present disclosure relates to a method of covalently attaching an adapter to a double stranded target polynucleotide comprising a 5' tail which terminates in a triplet nucleotide sequence selected from GGT, GGG, TGT, GTT, TGG, GGA, GTG, CGG, CGT and GCG. The inventors have shown that topoisomerase-activated sequencing adapters can covalently attach an adapter more readily, i.e. with greater efficiency, to 5' ends to double stranded polynucleotide target sequences having a 5' terminal triplet nucleotide sequence selected from GGT, GGG, TGT, GTT, TGG, GGA, GTG, CGG, CGT and GCG (see Example 1, Table A and Figure 3). Polynucleotide sequencing applications tend to be reliant upon target polynucleotides having a particular sequencing adapter in order for such target polynucleotides to be sequenced. Maximising the proportion of polynucleotides in a sample which comprise a sequencing adapter will in turn maximise the proportion of polynucleotides that are subject to sequencing. Accordingly, the method of the disclosure allows increased and/or improved sequencing information to be obtained from a polynucleotide sample.

Accordingly, the method of producing adapted amplicons described herein can be utilised to more efficiently covalently attach sequencing adapters to specific nucleic acid targets, and less efficiently to others. For example, amplification primers used in a multiplex PCR assay for the production of a sequencing library may result in varying levels of amplicons in a final library due to variable primer hybridisation, and resultant PCR amplification efficiencies. In this regard, upon identifying particular nucleic acid targets that are subject to decreased amplification relative to other targets in the sample (i.e. due to a decreased primer efficiency), such targets may be amplified using a first and second primer to produce a first amplicon, wherein at least one of the first and second primer comprises a 5' tail which terminates in a triplet nucleotide sequence selected from GGT, GGG, TGT, GTT, TGG, GGA, GTG, CGG, CGT and GCG. Having such a tail would mean that when the first amplicon is subject to step (ii), namely contacting the first amplicon with topoisomerase activated adapters such that the topoisomerase covalently links an adapter to the 5' end of the first amplicon, the triplet would lead to an increased efficiency of adapter attachment in the amplicon. As described above, increased efficiency of adapter attachment results in a greater proportion of that particular target sequence being sequenced, thus creating biases in observed amplicon representation when sequencing is carried out and thereby compensating for any observed decreased primer/amplification efficiency.

The inverse principle may be applied to particular nucleic acid targets that are subject to increased amplification relative to other targets in the sample. Such targets may be amplified using a third and fourth primer to produce an amplicon, wherein at least one of the third and fourth primer comprises a 5' tail which terminates in a triplet nucleotide sequence that is not GGT, GGG, TGT, GTT, TGG, GGA, GTG, CGG, CGT and GCG. Such triplet nucleotides include those other than the top ten in Table A and Figure 3, such as, for example, CAA, ATG, AAT, TAC, GCG, TAG, AAC, ATC, GCA, GCC, TAA, TTT, ATA, AAG, CCT, AAA, CCG, TTG, TTC, TCG, TCA, CCC, CCA, TCT, ACT, ACC, TCC, ACG, TTA or ACA. Having such a tail would mean that when the second amplicon is subject to step (ii), namely contacting the second amplicon with topoisomerase activated adapters such that the topoisomerase covalently links an adapter to the 5' end of the second amplicon, the triplet would lead to a decreased efficiency of adapter attachment in the amplicon. As described above, decreased efficiency of adapter attachment results in a decreased proportion of that particular target sequence being sequenced, thus creating biases in observed amplicon representation when sequencing is carried out and thereby compensating for an increased primer/amplification efficiency.

Amplification efficiencies of targets can be determined by any suitable method in the art. For large multiplex assays, sequencing analysis may preferably be used to determine amplification bias, and to determine whether the method of producing PCR adapted amplicons described herein may successfully modulate the amount of sequencing information derived from particular targets. Accordingly, the method may further comprise a step prior to (i) of determining, optionally by sequencing, whether a particular nucleic acid target has been disproportionately amplified during the preparation of a sequencing library. As described above, a nucleic acid target identified as having an increased or decreased amplification efficiency, as evidenced by, for example, disproportionately high or low sequencing reads, would then prompt the user to deploy steps (i) and (ii) in order to generate adapted PCR amplicons with variable adaptation efficiencies in order to compensate for the previously determined disproportionate amplification efficiency. The method may comprise a further step after (ii) to determine, optionally by sequencing, whether the method has successfully compensated for the observed increased or decreased amplification efficiencies of specific nucleic acid targets. The method of producing adapted PCR amplicons, optionally including the above described steps before (i) and after (ii), may be repeated any number of times deemed suitable in order to achieve a sufficient level of compensation for increased or decreased amplification efficiencies.

At least one of the first and second primers may comprise a 5' tail which terminates in the triplet nucleotide sequence GGT or GGG.

The 5' tail of the at least one of the third and fourth primers may terminate in a triplet nucleotide sequence selected from CAA, ATG, AAT, TAC, GCG, TAG, AAC, ATC, GCA, GCC, TAA, TTT, ATA, AAG, CCT, AAA, CCG, TTG, TTC, TCG, TCA, CCC, CCA, TCT, ACT, ACC, TCC, ACG, TTA or ACA.

The 5' tail of the at least one of the third and fourth primers may terminate in a triplet nucleotide sequence selected from ACA, TTA, ACG, TCC, ACC, ACT, TCT, CCA, CCC, TCA, TCG, TTC, TTG, CCG and AAA. Optionally, the third and fourth primers comprise a 5' tail which terminates in the triplet nucleotide sequence ACA.

In one embodiment, the 5' tail of the at least one of the first and second primers may terminate in a triplet nucleotide sequence selected from GGT or GGG, and the 5' tail of the at least one of the third and fourth primers may terminate in a triplet nucleotide sequence selected from ACA, TTA, ACG, TCC, ACC, ACT, TCT, CCA, CCC, TCA, TCG, TTC, TTG, CCG and AAA.

In one embodiment the 5' tail of the at least one of the first and second primers may terminate in a triplet nucleotide sequence selected from GGT or GGG, and the 5' tail of the at least one of the third and fourth primers may terminate in the triplet nucleotide sequence ACA.

### Kits

Also provided is a kit comprising
- a Vaccinia virus topoisomerase I
- a double stranded oligonucleotide adapter comprising a topoisomerase target site;
- a primer pair consisting of a first and second oligonucleotide primer,
wherein at least one of the first and second primers is a 5' tailed primer having a triplet nucleotide sequence selected from GGT, GGG, TGT, GTT, TGG, GGA, GTG, CGG, CGT and GCG at the 5' terminus of the tail.

Any of the topoisomerases described above with respect to any other aspect of the disclosure may be used in the kit.

Any of the double stranded oligonucleotide adapters described above with respect to any other aspect of the disclosure may be used in the kit. Preferably, the topoisomerase target site comprises a [C/T]CCTT nucleotide sequence. The adapter may further comprise a click reactive group, a fluorophore, a conjugation agent, a pull down group, a tethering moiety, a marker, a modified base, an abasic residue, a sequencing adapter, an intermediate adapter, an amplification adapter, a hairpin adapter, a unique molecular identifier, a helicase binding site and/or a spacer.

The Vaccinia virus topoisomerase I may be loaded on the double stranded oligonucleotide adapter, e.g. the adapter may be a topoisomerase activated adapter, such as described herein.

The primer pair, wherein at least one of the first and second primers is a 5' tailed primer having a triplet nucleotide sequence selected from GGT, GGG, TGT, GTT, TGG, GGA, GTG, CGG, CGT and GCG at the 5' terminus of the tail, may comprise the features of any of the primers described above with respect to any other aspect of the disclosure.

In the primer pair, at least one of the first and second primers is a 5' tailed primer having a triplet nucleotide sequence may be selected from GGT, GGG, TGT, GTT, TGG, GGA, GTG, CGG and CGT, or from GGT, GGG, TGT, GTT and TGG.

At least one of the first and second primers may comprise a 5' tail which terminates in the triplet nucleotide sequence GGT or GGG.

The kit may further comprise a primer pair consisting of a third oligonucleotide primer and a fourth oligonucleotide primer, wherein at least one of the third and fourth oligonucleotide primers is a 5' tailed primer which terminates in a triplet nucleotide sequence that is not GGT, GGG, TGT, GTT, TGG, GGA, GTG, CGG, CGT or GCG. Optionally, the 5' tail of the at least one of the third and fourth primers terminates in a triplet nucleotide sequence selected from CAA, ATG, AAT, TAC, GCG, TAG, AAC, ATC, GCA, GCC, TAA, TTT, ATA, AAG, CCT, AAA, CCG, TTG, TTC, TCG, TCA, CCC, CCA, TCT, ACT, ACC, TCC, ACG, TTA or ACA. Optionally, the 5' tail of the at least one of the third and fourth primers terminates in a triplet nucleotide sequence selected from ACA, TTA, ACG, TCC, ACC, ACT, TCT, CCA, CCC, TCA, TCG, TTC, TTG, CCG and AAA. Optionally, at least one of the third and fourth primers comprises a 5' tail which terminates in the triplet nucleotide sequence ACA.
The preceding embodiments and following examples are provided for illustration only, and should not be considered limiting the application. The application is limited only by the claims.

### EXAMPLES

The experiments detailed in the following Examples were carried out to investigate whether topoisomerase-activated sequencing adapters covalently attached to some amplicon 5' ends more readily than to others, thus creating biases in observed amplicon representation when sequencing was carried out.

### Example 1

This experiment was carried out to determine if the sequence at the 5' end of a target amplicon affected the downstream representation in sequencing of the amplicon, following topoisomerase-activated sequencing adapter attachment. PCR primers for three different target genes in *Mycobacterium tuberculosis* (TB) were generated; in each case, the primers were generated both with and without an additional 3-base sequence (NNN) appended to the 5' end of the primer (referred to as an NNN tail).

### Adapter preparation:

Two different types of topoisomerase adapter (Topo-adapter) were prepared: dT-tailed and blunt:

### dT-tailed:

DBCO-TEG/GCTTGGGTGTTTAACCAAAAGGTTACACAAACCCTGGACAA
GCCATATCCGTGTCGCCCTTATTCCGATAGTGACTACAGCGGCATGAGT
G

/5Phos/AGGGCGACACGGATATGGCTTGTCCAGGGTTTGTGTAACCTTTTm
UmUmUmUmU

/5Phos/TGTAGTCACTATCGGAAT

### Blunt:

DBCO-TEG/GCTTGGGTGTTTAACCAAAAGGTTACACAAACCCTGGACAA
GCCATATCCGTGTCGCCCTTATTCCGATAGTGACTACAGCGGCATGAGT
G

/5Phos/AAGGGCGACACGGATATGGCTTGTCCAGGGTTTGTGTAACCTTTT
mUmUmUmUmU

### /5Phos/TGTAGTCACTATCGGAAT

DBCO-TEG = click group to facilitate downstream attachment to sequencing adapter. mU = 2'-O-methyluridine.

Strand portions have the following functions shown by underline style:
No underline: overhang to attach to sequencing adapter.
Dotted underline: barcode.
Single underline: topoisomerase binding site, including CCCTT motif and flanking region.
Double underline: leaving group.

Adapters were prepared using the following method:
DNA adapters were generated by annealing strands at 10 µM in 10 mM Tris (pH7.5), 50 mM NaCl, from 95°C at 2°C per min.
Topo-DNA adapters were formed by mixing the following and incubating for 1 h at 37°C:
   4 µl nuclease-free H₂O
   1 µl 1M Tris (pH 7.5)
   5 µl DNA Adapter
   10 µl of Vaccinia Topoisomerase
   Following the 1 h incubation, add 20 µl of 50 mM Tris (pH 7.5), 100 mM NaCl, 5% glycerol.

### Primers:

The following primers were mixed to generate pairs of forward and reverse primers for three TB targets (*rpoB, gyrA* and *fabG1*) with or without NNN tails.

| Target | Control | |
|---|---|---|
| | Forward | Reverse |
| fabG1 | CTTTTGCACGCAATTGCGC | AGCAGTCCTGTCATGTGCG |
| gyrA | TGACAGACACGACGTTGCC | CGATCGCTAGCATGTTGGC |
| rpoB | TCATCATCAACGGGACCGAG | ACACGATCTCGTCGCTAACC |

| Target | NNN | |
|---|---|---|
| | Forward | Reverse |
| fabG1 | NNNCTTTTGCACGCAATTGCGC | NNNAGCAGTCCTGTCATGTGCG |
| gyrA | NNNTGACAGACACGACGTTGCC | NNNCGATCGCTAGCATGTTGGC |
| rpoB | NNNTCATCATCAACGGGACCGAG | NNNACACGATCTCGTCGCTAACC |

### PCR:

The following mixes were assembled for each of the six primer mixes;

| Reagent | Volume (µL) |
|---|---|
| water | 25.5 |
| Template | 2.5 |
| Primer mix (10 µM) | 2 |
| 5x Q soln | 20 |
| 2x QIAGEN MM | 50 |
| Total | 100 |

Amplification was carried out according to the following conditions:

| Step | Temp (°C) | Time | Cycles |
|---|---|---|---|
| Initial Denat | 95 | 15 m | 1 |
| Denat | 95 | 30 sec | 35 |
| Annealing | 60 | 1.5 min | |
| Extension | 72 | 1.5 min | |
| Final Extension | 72 | 10 m | 1 |
| Hold | 4 | | |

Samples were quantified using a Qubit system (ThermoFisher Scientific) and diluted to 25 ng/µL with water.

### Sequencing analysis:

The following reactions were assembled for control and NNN-tailed templates, and incubated for 10 min at 65°C and 2 min at 80°C.

| Reagent | Vol (µL) |
|---|---|
| Water | 30.5 |
| rpoB (25 ng/µL) | 4 |
| gyrA (25 ng/µL) | 4 |
| fabG1 (25 ng/µL) | 4 |
| 500 mM EDTA | 2.5 |
| dT topo adapter | 2.5 |
| Blunt topo adapter | 2.5 |
| Final | 50 |

The following steps were carried out:
- SPRI purify (0.7x), wash 2x with 70% EtOH and elute in 14 µL EB.
- Seq adapter addition: to 11 µL of eluted library, add 1 µL of RAP-F rapid sequencing adapter, and incubate for 10 mins at RT before running on MinION sequencing device (Oxford Nanopore Technologies) for 12 hours.

Total read counts were measured. Reads were then down-sampled and aligned to the TB genome and read counts for each target (in forward and reverse orientation) were recorded.

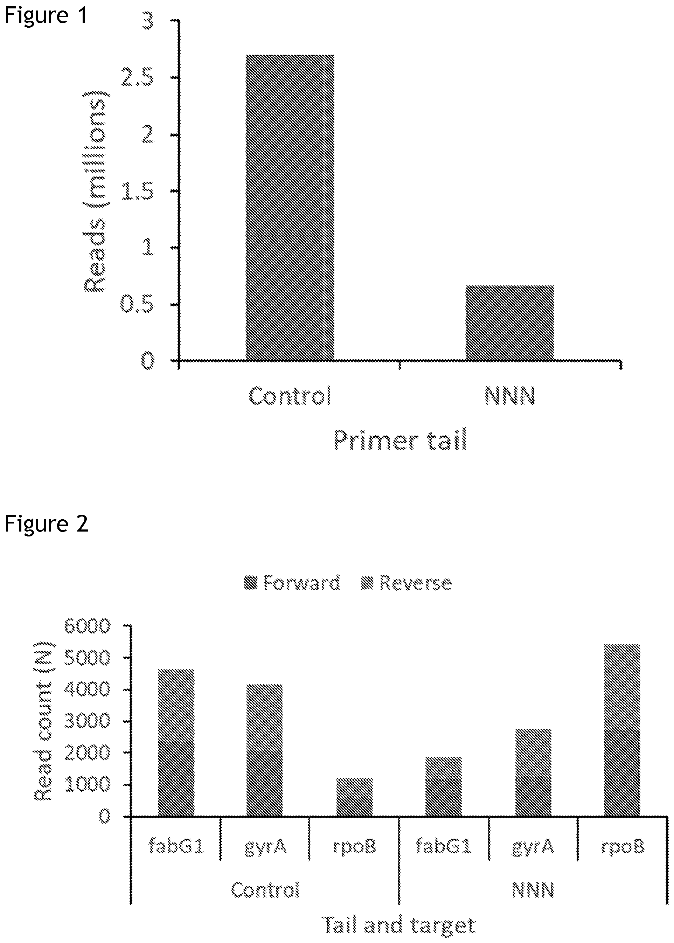

It was noted that altering the 5' tail sequence on the primers changed the sequencing performance, with overall sequencing performance decreased. This is shown in Figure 1 by the overall reduction in total reads for amplicons obtained with NNN-tailed primers compared to amplicons obtained with control primers, showing that the use of NNN-tailed primers reduced topoisomerase-mediated sequencing adapter attachment to these amplicons.

Figure 2 shows total read counts obtained following sequencing on Oxford Nanopore Technologies MinION sequencing device of amplicons obtained in Example 1 using control and NNN-tailed primers (control compared to NNN-tailed for individual target genes).

It was further noted that the performance of individual amplicons was also altered; notably the representation of *rpoB* was increased when using NNN-tailed primers compared to the control.

As multiple different NNN sequences had been tested, the frequency at which each trimer sequence in the NNN motif was sequenced was determined. Read counts for each different trimer sequence are shown in Table A, with results presented for individual target genes (Forward (F) and Reverse (R)) and all results combined.

**Table A**

| | **fabG1_F** | **fabG1_R** | **gyrA_F** | **gyrA_R** | **rpoB_F** | **rpoB_R** | **Combined** |
|---|---|---|---|---|---|---|---|
| GGT | 2065 | 904 | 1969 | 2342 | 3190 | 2979 | 13449 |
| GGG | 2260 | 592 | 3094 | 1508 | 2907 | 2821 | 13182 |
| TGT | 1069 | 489 | 798 | 1307 | 2750 | 2320 | 8733 |
| GTT | 1058 | 707 | 713 | 1197 | 2879 | 1803 | 8357 |
| TGG | 1717 | 466 | 736 | 1204 | 1930 | 1811 | 7864 |
| GGA | 1221 | 377 | 876 | 1605 | 1713 | 1305 | 7097 |
| GTG | 1223 | 530 | 705 | 1479 | 1876 | 1214 | 7027 |
| CGG | 875 | 361 | 934 | 1264 | 1572 | 1884 | 6890 |
| CGT | 564 | 464 | 780 | 1005 | 2042 | 1953 | 6808 |
| GGC | 718 | 286 | 971 | 1476 | 1695 | 1082 | 6228 |
| TGC | 434 | 310 | 389 | 666 | 1303 | 1158 | 4260 |
| GAT | 628 | 301 | 364 | 777 | 1273 | 848 | 4191 |
| TGA | 609 | 213 | 314 | 705 | 1174 | 1038 | 4053 |
| GTA | 551 | 288 | 245 | 673 | 1196 | 924 | 3877 |
| CGC | 297 | 263 | 373 | 731 | 1043 | 1051 | 3758 |
| CTT | 282 | 173 | 212 | 397 | 1572 | 1084 | 3720 |
| CGA | 340 | 222 | 366 | 673 | 1035 | 1004 | 3640 |
| GAG | 1004 | 151 | 309 | 661 | 848 | 665 | 3638 |
| GTC | 424 | 294 | 309 | 563 | 1197 | 849 | 3636 |
| AGT | 528 | 212 | 312 | 551 | 1167 | 855 | 3625 |
| CAT | 229 | 164 | 205 | 442 | 1077 | 772 | 2889 |
| CTG | 260 | 148 | 143 | 403 | 1195 | 667 | 2816 |
| AGG | 645 | 134 | 212 | 360 | 782 | 589 | 2722 |
| GAC | 254 | 193 | 276 | 522 | 895 | 444 | 2584 |
| GAA | 318 | 106 | 235 | 531 | 784 | 543 | 2517 |
| ATT | 274 | 131 | 102 | 266 | 759 | 729 | 2261 |
| CAG | 256 | 129 | 138 | 334 | 719 | 591 | 2167 |
| TAT | 199 | 96 | 97 | 273 | 834 | 567 | 2066 |
| CTA | 130 | 71 | 84 | 235 | 767 | 745 | 2032 |
| AGC | 275 | 176 | 140 | 465 | 498 | 403 | 1957 |
| CTC | 121 | 99 | 117 | 234 | 722 | 660 | 1953 |
| GCT | 266 | 105 | 88 | 365 | 620 | 461 | 1905 |
| AGA | 242 | 92 | 157 | 346 | 645 | 419 | 1901 |
| CAC | 114 | 148 | 193 | 305 | 599 | 377 | 1736 |
| CAA | 111 | 98 | 107 | 265 | 613 | 532 | 1726 |
| ATG | 181 | 73 | 104 | 169 | 760 | 407 | 1694 |
| AAT | 165 | 93 | 82 | 212 | 677 | 459 | 1688 |
| TAC | 144 | 119 | 109 | 245 | 666 | 398 | 1681 |
| GCG | 260 | 77 | 142 | 365 | 476 | 330 | 1650 |
| TAG | 252 | 59 | 65 | 206 | 539 | 296 | 1417 |
| AAC | 114 | 75 | 96 | 183 | 548 | 384 | 1400 |
| ATC | 117 | 56 | 62 | 127 | 593 | 365 | 1320 |
| GCA | 152 | 66 | 62 | 229 | 444 | 343 | 1296 |
| GCC | 99 | 115 | 80 | 209 | 465 | 294 | 1262 |
| TAA | 132 | 48 | 60 | 183 | 417 | 380 | 1220 |
| TTT | 121 | 67 | 45 | 75 | 373 | 445 | 1126 |
| ATA | 111 | 52 | 41 | 95 | 427 | 277 | 1003 |
| AAG | 153 | 32 | 52 | 161 | 332 | 222 | 952 |
| CCT | 80 | 44 | 29 | 102 | 358 | 266 | 879 |
| AAA | 93 | 26 | 46 | 99 | 306 | 215 | 785 |
| CCG | 86 | 39 | 47 | 125 | 227 | 241 | 765 |
| TTG | 87 | 28 | 30 | 79 | 267 | 217 | 708 |
| TTC | 41 | 29 | 30 | 34 | 258 | 251 | 643 |
| TCG | 91 | 29 | 25 | 99 | 162 | 215 | 621 |
| TCA | 65 | 17 | 22 | 48 | 260 | 183 | 595 |
| CCC | 33 | 35 | 31 | 85 | 258 | 148 | 590 |
| CCA | 35 | 29 | 25 | 66 | 273 | 157 | 585 |
| TCT | 63 | 27 | 17 | 58 | 256 | 160 | 581 |
| ACT | 57 | 37 | 12 | 50 | 218 | 181 | 555 |
| ACC | 27 | 36 | 29 | 47 | 226 | 150 | 515 |
| TCC | 19 | 32 | 16 | 49 | 196 | 148 | 460 |
| ACG | 64 | 27 | 25 | 58 | 152 | 99 | 425 |
| TTA | 49 | 21 | 10 | 41 | 152 | 148 | 421 |
| ACA | 30 | 20 | 14 | 37 | 146 | 103 | 350 |

Figure 3 shows a graph of the date presented in Table A, showing total Read Counts obtained with each NNN motif for all six targets.

It was observed that certain motifs were observed more frequently than others, and thus associated with higher read counts than others. The ten motifs shown to be associated with the highest read counts in Table A are the triplet nucleotide sequences useful in the methods provided herein. In particular, the motifs GGT, GGG, TGT, GTT, TGG, GGA, GTG, CGG, CGT and GCG were associated with the highest combined read counts (shown on the left-hand side of the graph in Figure 3).

The most common motifs that were observed were GGT and GGG (GGK, where K represents G or T). The least frequent was ACA. The ten motifs shown to be associated with the highest read counts in Table A are, when present at the 5' end of one or both strands the plurality of double stranded target polynucleotides in the methods herein, the most susceptible to modification by the topo adaptor. Thus, targets having a motif selected from GGT, GGG, TGT, GTT, TGG, GGA, GTG, CGG, CGT and GCG, and in particular GGT or GGG (GGK), at the 5' end are more susceptible to modification by the topo adapter.

The motifs shown to be associated with the lowest read counts in Table A are, when present at the 5' end of one or both strands of double stranded target polynucleotides, the least susceptible to modification by the topo adaptor. Thus, targets having a motif selected from CAA, ATG, AAT, TAC, GCG, TAG, AAC, ATC, GCA, GCC, TAA, TTT, ATA, AAG, CCT, AAA, CCG, TTG, TTC, TCG, TCA, CCC, CCA, TCT, ACT, ACC, TCC, ACG, TTA and ACA, at the 5' end are less susceptible to modification by the topo adapter. Targets having a motif selected from ACA, TTA, ACG, TCC, ACC, ACT, TCT, CCA, CCC, TCA, TCG, TTC, TTG, CCG and AAA are even less susceptible. The triplet sequence ACA is the least susceptible.

### Example 2

This experiment was carried out to determine whether the presence of a GGK tail improved amplicon representation (by generating a more even modification across amplicons by the topo adapters) and improved sequencing outputs, using the same TB target genes as in Example 1.

### Primers:

The following primers were mixed to generate pairs of forward and reverse primers for three TB targets *(rpoB, gyrA* and *fabG1*) with or without GGK tails. (In this Example and the Examples below, primers indicated as "GGK" primers having a GGK tail comprise a mixture of GGG and GGT tails obtained during primer manufacture.)

| Target | Control | |
|---|---|---|
| | Forward | Reverse |
| fabG1 | CTTTTGCACGCAATTGCGC | AGCAGTCCTGTCATGTGCG |
| gyrA | TGACAGACACGACGTTGCC | CGATCGCTAGCATGTTGGC |
| rpoB | TCATCATCAACGGGACCGAG | ACACGATCTCGTCGCTAACC |

| Target | GGK | |
|---|---|---|
| | Forward | Reverse |
| fabG1 | GGKCTTTTGCACGCAATTGCGC | GGKAGCAGTCCTGTCATGTGCG |
| gyrA | GGKTGACAGACACGACGTTGCC | GGKCGATCGCTAGCATGTTGGC |
| rpoB | GGKTCATCATCAACGGGACCGAG | GGKACACGATCTCGTCGCTAACC |

### PCR:

The following mixes were assembled for each of the six primer mixes:

| Reagent | Volume (µL) |
|---|---|
| water | 25.5 |
| Template | 2.5 |
| Primer mix (10 µM) | 2 |
| 5x Q soln | 20 |
| 2x QIAGEN MM | 50 |
| Total | 100 |

Amplification was carried out according to the following conditions:

| Step | Temp (°C) | Time | Cycles |
|---|---|---|---|
| Initial Denat | 95 | 15 m | 1 |
| Denat | 95 | 30 sec | 35 |
| Annealing | 60 | 1.5 min | |
| Extension | 72 | 1.5 min | |
| Final Extension | 72 | 10 m | 1 |
| Hold | 4 | | |

Samples were quantified using a Qubit system (ThermoFisher Scientific) and diluted to 25 ng/µL with water.

### Agilent analysis:

The following reactions were assembled for each of the six amplicons. Incubation was carried out for 10 mins at 65°C and 2 mins at 80°C. Topo-adapter was prepared according to the methods set out in Example 1.

| Reagent | -ve | +ve |
|---|---|---|
| Water | 5 | 4 |
| 50 mM EDTA | 4 | 4 |
| Template (25 ng/uL) | 1 | 1 |
| dT topo adapter | | 1 |
| Final | 10 | 10 |

2 µL samples of each were run on an Agilent 2100 Bioanalyzer 12000 chip.

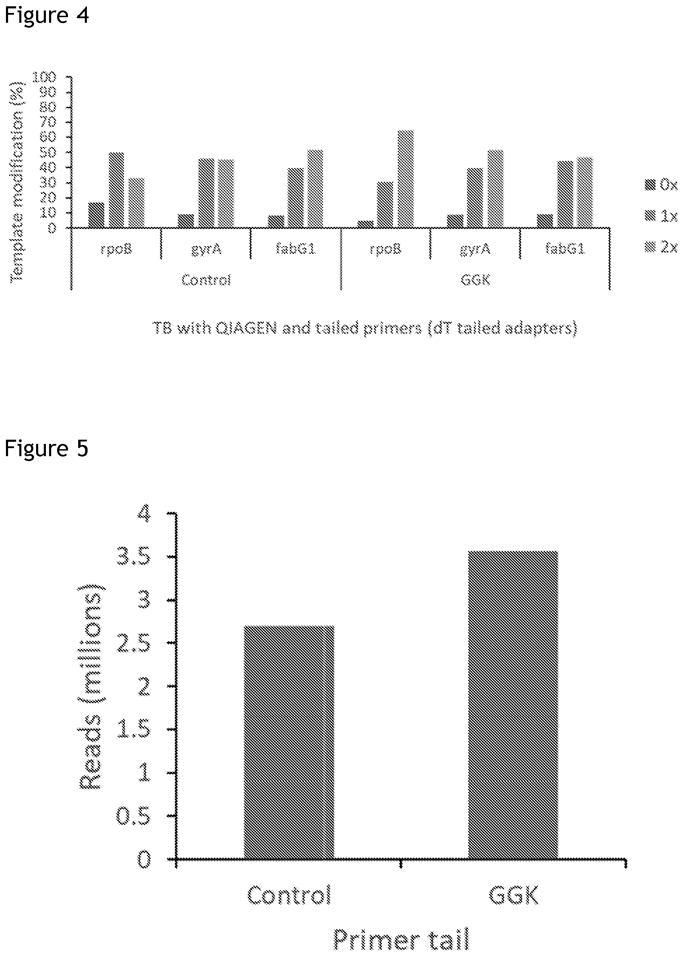

Modification by topo-adapter was significantly improved for rpoB by the addition of a GGK tail. Modification of gyrA was also improved (Figure 4).

Figure 4 shows percentage template modification for the three different targets for Control and GGK tails. 0x = no adapter attached; 1x = adapter attached to one end of amplicon; 2x = adapters attached to both ends of amplicon; shown left to right for each target.

### Sequencing analysis:

The following reactions were assembled for control and NNN-tailed templates. Incubation was carried out for 10 mins at 65°C and 2 mins at 80°C.

| Reagent | Vol (µL) |
|---|---|
| Water | 30.5 |
| rpoB (25 ng/µL) | 4 |
| gyrA (25 ng/µL) | 4 |
| fabG1 (25 ng/µL) | 4 |
| 500 mM EDTA | 2.5 |
| dT topo adapter | 2.5 |
| Blunt topo adapter | 2.5 |
| Final | 50 |

The following steps were carried out:
- SPRI purify (0.7x), wash 2x with 70% EtOH and elute in 14 µL EB.
- Seq adapter addition: to 11 µL of eluted library, add 1 µL of RAP-F rapid sequencing adapter, and incubate for 10 mins at RT before running on MinION sequencing device (Oxford Nanopore Technologies) for 12 hours.

Total read counts were measured. Reads were then down-sampled and aligned to the TB genome and read counts for each target (in forward and reverse orientation) were recorded.

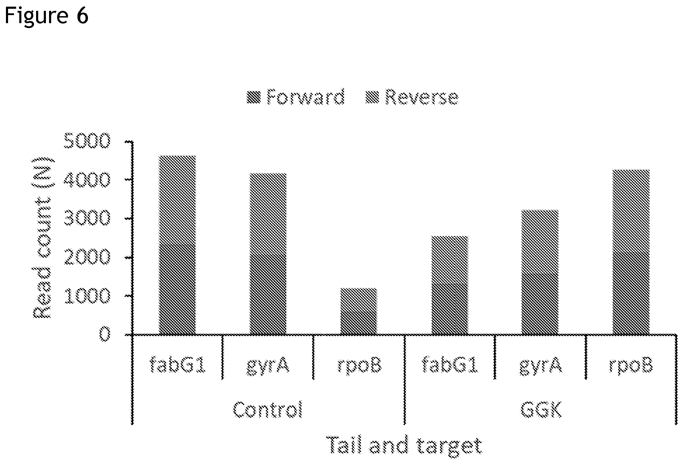

Changing the tail on the end of the primers to GGK improved the sequencing performance (increased read count). The performance of individual amplicons was also altered, with representation being more even across the three different targets. Figure 5 shows read counts obtained with control and GGK-tailed amplicons. Figure 6 shows read counts obtained showing control and GGK-tailed amplicons for three target genes.

### Example 3

This experiment was carried out to determine whether the presence of a GGK tail improved modification by topo-adapters, with an additional benefit of flattening amplicon representation and improving sequencing outputs, using target sequences from *Escherichia coli.*

### Primers:

The following primers were mixed to generate pairs of forward and reverse primers for 12 *E. coli* targets with or without GGK tails.

| Target | Control | |
|---|---|---|
| | Forward | Reverse |
| 1 | CGAAGGCATGAGTTTCTCCG | CGAAGGCATGAGTTTCTCCG |
| 2 | GACGAGATCCTGAAACGTGC | GACGAGATCCTGAAACGTGC |
| 3 | GTGCTGGAAATCGTGCGTAT | GTGCTGGAAATCGTGCGTAT |
| 4 | CCCATAAGACGTCAGCAAGC | CCCATAAGACGTCAGCAAGC |
| 5 | CAACATCATCCTCGCACCAG | CAACATCATCCTCGCACCAG |
| 6 | TTACGGTTCATCAGCATGCG | TTACGGTTCATCAGCATGCG |
| 7 | CACTGCCTGGCCGTAAATAC | CACTGCCTGGCCGTAAATAC |
| 8 | CGTGGATTAAGCAGGTGAGC | CGTGGATTAAGCAGGTGAGC |
| 9 | TTCCCTGCTGCTCAATACCA | TTCCCTGCTGCTCAATACCA |
| 10 | CGAATCCGCCTGCAACATAA | CGAATCCGCCTGCAACATAA |
| 11 | GTGAATACTTCCTGCCGCAG | GTGAATACTTCCTGCCGCAG |
| 12 | GCCGTAATGTTAAGCGCAGA | GCCGTAATGTTAAGCGCAGA |

| Target | GGK | |
|---|---|---|
| | Forward | Reverse |
| 1 | GGKCGAAGGCATGAGTTTCTCCG | GGKTGTCGTAATGAATGCTGCCG |
| 2 | GGKGACGAGATCCTGAAACGTGC | GGKTCAGGTATTTGCTGCCCAGA |
| 3 | GGKGTGCTGGAAATCGTGCGTAT | GGKACGCGTTCTTTATCGCCTTC |
| 4 | GGKCCCATAAGACGTCAGCAAGC | GGKGGTCATAAAACGGCGCTGAT |
| 5 | GGKCAACATCATCCTCGCACCAG | GGKCAAAGAATCGCCGGAAGAGG |
| 6 | GGKTTACGGTTCATCAGCATGCG | GGKAGATGGGGCAGAAAATGGGA |
| 7 | GGKCACTGCCTGGCCGTAAATAC | GGKGTTGATGCGCTCGGTAATGT |
| 8 | GGKCGTGGATTAAGCAGGTGAGC | GGKGATCACATGCAGCACACGAT |
| 9 | GGKTTCCCTGCTGCTCAATACCA | GGKTTGGTCCGATTACTGGGCTT |
| 10 | GGKCGAATCCGCCTGCAACATAA | GGKGCAGAAATCCGCGAACAGAT |
| 11 | GGKGTGAATACTTCCTGCCGCAG | GGKCCAGTTGATGATGGTGAGCG |
| 12 | GGKGCCGTAATGTTAAGCGCAGA | GGKTGGATGAACTGCAGGATGGT |

### PCR:

- Each of the 24 targets was amplified using NEB (New England Biolabs) Hot Start Master Mix according to the manufacturer's instructions.
- Samples were quantified using a Qubit system (ThermoFisher Scientific) and diluted to 25 ng/µL with water.

### Agilent analysis:

The following reactions were assembled for each of the 24 amplicons. Incubation was carried out for 10 mins at 65°C and 2 mins at 80°C. Topo-adapter was prepared according to the methods set out in Example 1.

| Reagent | -ve | +ve |
|---|---|---|
| Water | 5 | 4 |
| 50 mM EDTA | 4 | 4 |
| Template (25 ng/uL) | 1 | 1 |
| Blunt topo adapter | | 1 |
| Final | 10 | 10 |

2 µL samples of each were run on an Agilent 2100 Bioanalyzer 12000 chip.

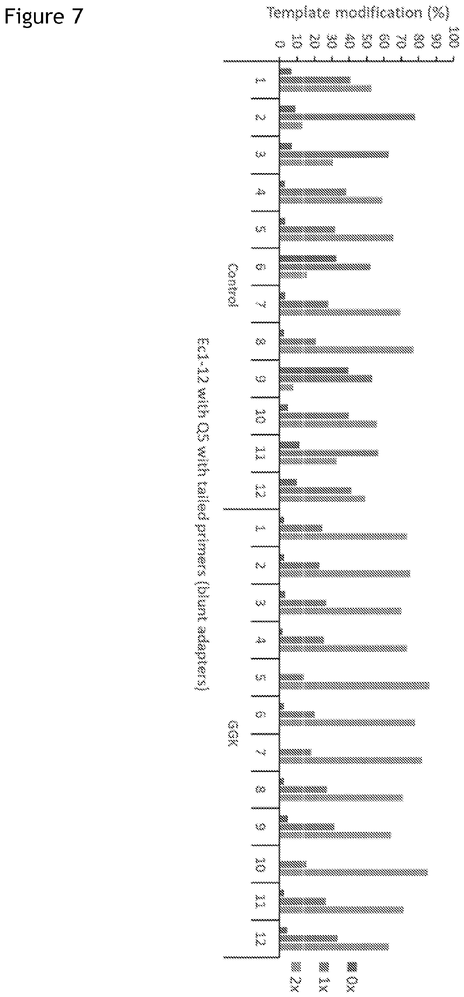

Modification by topo-adapter was significantly improved for the majority of targets that comprised GGK tails. Amplicons 2, 6 and 9 in particular showed very large improvements. (Figure 7). Figure 7 shows percentage template modification for the 12 different targets for control and GGK tails. 0x = no adapter attached; 1x = adapter attached to one end of amplicon; 2x = adapters attached to both ends of amplicon; shown left to right for each target.

### Sequencing analysis:

- Tailed or untailed amplicons were mixed and then modified using blunt topo adapters by incubating for 10 mins at 65°C and 2 mins at 80°C.
- The topo modified pool was SPRI purified (0.7x), washed 2x with 70% EtOH and eluted in 14 µL EB.
- Sequencing adapter addition: to 11 µL of eluted library, add 1 µL of RAP-F, and incubate for 10 mins at RT before running on MinION sequencing device.

Reads were down-sampled and aligned to the *E.coli* genome and read counts for each target (in forward and reverse orientation) were recorded.

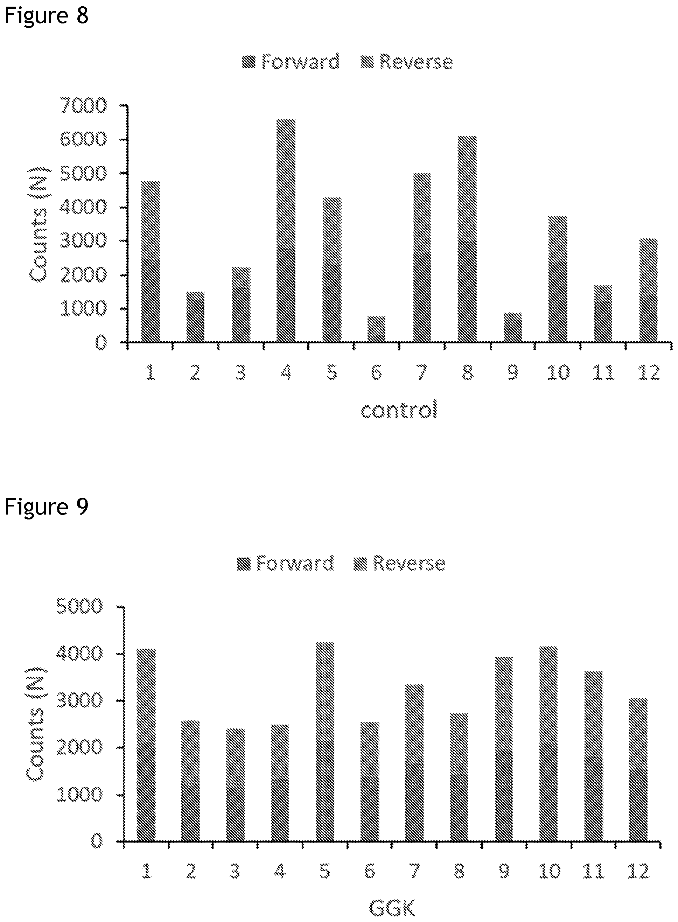

In non-tailed samples, amplicon representation was highly variable; low representation of amplicons 2, 6 and 9 in particular was observed (matching their poor levels of topo modification); also observed were biases in the forward/reverse orientation. (Figure 8). Figure 8 shows read count for *E. coli* targets, control amplicons.

The addition of GGK tails made the overall representation of the targets much flatter, with a much more even split between forward and reverse reads. (Figure 9). Figure 9 shows read count for *E. coli* targets, GGK-tailed amplicons.

### Example 4

This experiment was carried out to determine whether the presence of ACA tails reduced modification by topo-adapters.

### Primers:

The following primers were mixed to generate pairs of forward and reverse primers for *E. coli* target 1 (from Example 3) using GGK tail, ACA tail, or no tail.

| Target | Control | |
|---|---|---|
| | Forward | Reverse |
| 1 | CGAAGGCATGAGTTTCTCCG | CGAAGGCATGAGTTTCTCCG |
| 1_GGK | GGKCGAAGGCATGAGTTTCTCCG | GGKTGTCGTAATGAATGCTGCCG |
| 1_ACA | ACACGAAGGCATGAGTTTCTCCG | ACATGTCGTAATGAATGCTGCCG |

### PCR:

- Each of the three targets was amplified using NEB Q5 Hot Start Master Mix according to manufacturer's instructions.
- Samples were quantified using a Qubit system (ThermoFisher Scientific) and diluted to 25 ng/µL with water.

### Agilent analysis:

The following reactions were assembled for each of the 3 amplicons. Incubation was carried out for 10 mins at 65°C and 2 mins at 80°C. Topo-adapter was prepared according to the methods set out in Example 1.

| Reagent | -ve | +ve |
|---|---|---|
| Water | 5 | 4 |
| 50 mM EDTA | 4 | 4 |
| Template (25 ng/uL) | 1 | 1 |
| Blunt topo adapter | | 1 |
| Final | 10 | 10 |

2 µL samples of each were run on an Agilent 2100 Bioanalyzer 12000 chip.

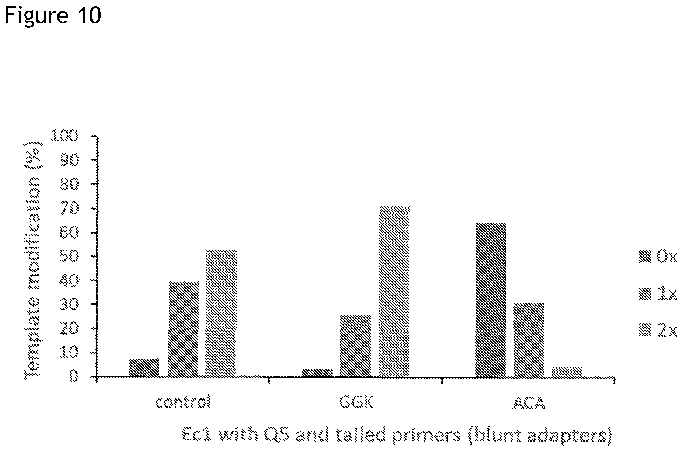

Modification by topoisomerase was improved by the addition of a GGK tail but was severely impeded by the addition of an ACA tail (Figure 10). Figure 10 shows percentage template modification for Control, GGK, and ACA tails. 0x = no adapter attached; 1x = adapter attached to one end of amplicon; 2x = adapters attached to both ends of amplicon; shown left to right for each target.

## Claims

1. A method of preparing a library of nucleic acids, the method comprising:
(a) providing a plurality of topoisomerase activated adapters, each comprising a Vaccinia virus topoisomerase I bound to a double stranded oligonucleotide;
(b) providing a plurality of double stranded target polynucleotides, each comprising a triplet nucleotide sequence at the 5' end of one or both strands, wherein the triplet nucleotide sequence is selected from GGT, GGG, TGT, GTT, TGG, GGA, GTG, CGG, CGT and GGC; and
(c) contacting the topoisomerase activated adapters with the plurality of double stranded target polynucleotides, such that the topoisomerase covalently links the activated adapters to the 5' ends of the double stranded target polynucleotides, thereby preparing a library of nucleic acids.

2. The method according to claim 1, which further comprises producing the plurality of double stranded target polynucleotides comprising the triplet nucleotide sequence at the 5' end of one or both strands by PCR using a first oligonucleotide primer and a second oligonucleotide primer, wherein at least one of the first and second primers comprises a 5' tail which terminates in the triplet nucleotide sequence.

3. The method according to claim 2, wherein both the first and second oligonucleotide primers comprise a 5' tail which terminates in the triplet nucleotide sequence.

4. The method according to any one of claims 1 to 3, wherein the adapters are sequencing adapters.

5. The method according to any one of claims 1 to 4, wherein the triplet nucleotide sequence is GGT or GGG.

6. The method according to any one of claims 1 to 5, wherein the triplet nucleotide sequence at the 5' end of one or both strands of the plurality of double stranded target polynucleotides is immediately 5' to a second triplet nucleotide sequence selected from GGT, GGG, TGT, GTT, TGG, GGA, GTG, CGG, CGT and GGC, optionally wherein the second triplet nucleotide sequence at the 5' end of one or both strands of the plurality of double stranded target polynucleotides is immediately 5' to a third triplet nucleotide sequence selected from GGT, GGG, TGT, GTT, TGG, GGA, GTG, CGG, CGT and GGC.

7. The method according to any one of claims 1 to 6, wherein:
i. the double stranded target polynucleotides are blunt-ended; or
ii. the double stranded target polynucleotides comprise a nucleotide overhang. optionally wherein the double stranded target polynucleotides are A-tailed.

8. The method according to any one of claims 1 to 7, wherein the adapter further comprises a click reactive group, a fluorophore, a conjugation agent, a pull down group, a tethering moiety, a marker, a modified base, an abasic residue, an intermediate adapter, an amplification adapter, a hairpin adapter, a unique molecular identifier, a helicase binding site and/or a spacer.

9. The method according to any one of claims 1 to 8, further comprising determining the presence, absence or one or more characteristics of the double stranded target polynucleotides covalently linked to the adapters, the method comprising:
(a) contacting the target polynucleotide with a nanopore or a mutant thereof such that the target polynucleotide moves with respect to the pore; and
(b) taking one or more measurements as the polynucleotide moves with respect to the pore,
thereby determining the presence, absence or one or more characteristics of the polynucleotide.

10. A method of attaching a polynucleotide adapter to a double stranded target polynucleotide, the method comprising:
(a) providing a topoisomerase activated adapter comprising a Vaccinia virus topoisomerase I bound to a double stranded oligonucleotide;
(b) providing a double stranded target polynucleotide comprising a triplet nucleotide sequence at the 5' end of one or both strands, wherein the triplet nucleotide sequence is selected from GGT, GGG, TGT, GTT, TGG, GGA, GTG, CGG, CGT and GGC; and
(c) contacting the topoisomerase activated adapter with the double stranded target polynucleotide such that the topoisomerase covalently links the activated adapter to the 5' ends of the double stranded target polynucleotide,
thereby attaching a polynucleotide adapter to a double stranded target polynucleotide.

11. A method of producing adapted PCR amplicons, the method comprising:
(i) amplifying a first nucleic acid using a first oligonucleotide primer and a second oligonucleotide primer to produce a first amplicon, wherein at least one of the first and second primers comprises a 5' tail which terminates in a triplet nucleotide sequence selected from GGT, GGG, TGT, GTT, TGG, GGA, GTG, CGG, CGT and GGC and amplifying a second nucleic acid using a third primer and a fourth primer to produce a second amplicon, wherein at least one of the third and fourth primers comprises a 5' tail which terminates in a triplet nucleotide sequence that is not GGT, GGG, TGT, GTT, TGG, GGA, GTG, CGG, CGT or GGC; and
(ii) contacting the first amplicon and the second amplicon with topoisomerase activated adapters comprising a Vaccinia virus topoisomerase I bound to an adapter such that the topoisomerase covalently links an adapter to the 5' end of the first amplicon and to the 5' end of the second amplicon.

12. The method according to claim 11, wherein:
(i) at least one of the first and second primers comprises a 5' tail which terminates in the triplet nucleotide sequence GGT or GGG; and/or
(ii) the 5' tail of the at least one of the third and fourth primers terminates in a triplet nucleotide sequence selected from ACA, TTA, ACG, TCC, ACC, ACT, TCT, CCA, CCC, TCA, TCG, TTC, TTG, CCG and AAA, optionally at least one of the third and fourth primers comprises a 5' tail which terminates in the triplet nucleotide sequence ACA.

13. A kit comprising:
- a Vaccinia virus topoisomerase I;
- a double stranded oligonucleotide adapter comprising a topoisomerase target site;
- a primer pair consisting of a first and second oligonucleotide primer, wherein at least one of the first and second primers is a 5' tailed primer having a triplet nucleotide sequence selected from GGT, GGG, TGT, GTT, TGG, GGA, GTG, CGG, CGT and GGC at the 5' terminus of the tail.

14. The kit according to claim 13, wherein:
(i) at least one of the first and second primers comprises a 5' tail which terminates in the triplet nucleotide sequence GGT or GGG;
(ii) the kit further comprises a primer pair consisting of a third oligonucleotide primer and a fourth oligonucleotide primer, wherein at least one of the third and fourth oligonucleotide primers is a 5' tailed primer which terminates in a triplet nucleotide sequence that is not GGT, GGG, TGT, GTT, TGG, GGA, GTG, CGG, CGT or GGC, optionally wherein the 5' tail of the at least one of the third and fourth primers terminates in a triplet nucleotide sequence selected from ACA, TTA, ACG, TCC, ACC, ACT, TCT, CCA, CCC, TCA, TCG, TTC, TTG, CCG and AAA, and further optionally wherein at least one of the third and fourth primers comprises a 5' tail which terminates in the triplet nucleotide sequence ACA.

15. The kit according to any claim 13 or claim 14, wherein the topoisomerase is loaded on the adapter and/or the adapter further comprises a click reactive group, a fluorophore, a conjugation agent, a pull down group, a tethering moiety, a marker, a modified base, an abasic residue, an intermediate adapter, an amplification adapter, a hairpin adapter, a unique molecular identifier, a helicase binding site and/or a spacer.

## Patentansprüche

1. Verfahren zum Herstellen einer Bibliothek von Nukleinsäuren, das Verfahren umfassend:
(a) Bereitstellen einer Vielzahl von Topoisomerase-aktivierten Adaptern, umfassend jeweils eine an ein doppelsträngiges Oligonukleotid gebundene Vaccinia-Virus-Topoisomerase I;
(b) Bereitstellen einer Vielzahl von doppelsträngigen Zielpolynukleotiden, jeweils umfassend eine Triplett-Nukleotidsequenz am 5'-Ende eines oder beider Stränge, wobei die Triplett-Nukleotidsequenz aus GGT, GGG, TGT, GTT, TGG, GGA, GTG, CGG, CGT und GGC ausgewählt ist; und
(c) Inkontaktbringen der Topoisomerase-aktivierten Adapter mit der Vielzahl von doppelsträngigen Zielpolynukleotiden, sodass die Topoisomerase die aktivierten Adapter kovalent an die 5'-Enden der doppelsträngigen Zielpolynukleotide bindet, wodurch eine Bibliothek von Nukleinsäuren hergestellt wird.

2. Verfahren nach Anspruch 1, das ferner das Herstellen der Vielzahl von doppelsträngigen Zielpolynukleotiden, umfassend die Triplett-Nukleotidsequenz am 5'-Ende eines oder beider Stränge, durch PCR unter Verwendung eines ersten Oligonukleotid-Primers und eines zweiten Oligonukleotid-Primers umfasst, wobei mindestens einer der ersten und zweiten Primer einen 5'-Schwanz umfasst, der in der Triplett-Nukleotidsequenz endet.

3. Verfahren nach Anspruch 2, wobei sowohl der erste als auch der zweite Oligonukleotid-Primer einen 5'-Schwanz umfassen, der in der Triplett-Nukleotidsequenz endet.

4. Verfahren nach einem der Ansprüche 1 bis 3, wobei es sich bei den Adaptern um Sequenzierungsadapter handelt.

5. Verfahren nach einem der Ansprüche 1 bis 4, wobei es sich bei der Triplett-Nukleotidsequenz um GGT oder GGG handelt.

6. Verfahren nach einem der Ansprüche 1 bis 5, wobei die Triplett-Nukleotidsequenz am 5'-Ende eines oder beider Stränge der Vielzahl von doppelsträngigen Zielpolynukleotide unmittelbar 5' zu einer zweiten Triplett-Nukleotidsequenz liegt, die aus GGT, GGG, TGT, GTT, TGG, GGA, GTG, CGG, CGT und GGC ausgewählt ist, wobei optional die zweite Triplett-Nukleotidsequenz am 5'-Ende von einem oder beiden Strängen der Vielzahl von doppelsträngigen Zielpolynukleotiden unmittelbar 5' zu einer dritten Triplett-Nukleotidsequenz liegt, die aus GGT, GGG, TGT, GTT, TGG, GGA, GTG, CGG, CGT und GGC ausgewählt ist.

7. Verfahren nach einem der Ansprüche 1 bis 6, wobei:
i. die doppelsträngigen Zielpolynukleotide stumpf endend sind; oder
ii. die doppelsträngigen Zielpolynukleotide einen Nukleotidüberhang umfassen, wobei die doppelsträngigen Zielpolynukleotide optional A-geschwänzt sind.

8. Verfahren nach einem der Ansprüche 1 bis 7, wobei der Adapter ferner eine Click-reaktive Gruppe, ein Fluorophor, ein Konjugationsmittel, eine Pull-Down-Gruppe, eine Tethering-Gruppe, einen Marker, eine modifizierte Base, einen basischen Rest, einen Zwischenadapter, einen Amplifikationsadapter, einen Haarnadeladapter, einen eindeutigen molekularen Identifikator, eine Helikase-Bindungsstelle und/oder einen Spacer umfasst.

9. Verfahren nach einem der Ansprüche 1 bis 8, ferner umfassend das Bestimmen des Vorhandenseins, der Abwesenheit oder eines oder mehrerer Merkmale der doppelsträngigen Zielpolynukleotide, die kovalent mit den Adaptern verbunden sind, das Verfahren umfassend:
(a) Inkontaktbringen des Zielpolynukleotids mit einer Nanopore oder einer Mutante davon, sodass sich das Zielpolynukleotid in Bezug auf die Pore bewegt; und
(b) Durchführen einer oder mehrerer Messungen, während sich das Polynukleotid in Bezug auf die Pore bewegt, wodurch das Vorhandensein, die Abwesenheit oder ein oder mehrere Merkmale des Polynukleotids bestimmt werden.

10. Verfahren zum Binden eines Polynukleotid-Adapters an ein doppelsträngiges Zielpolynukleotid, das Verfahren umfassend:
(a) Bereitstellen eines Topoisomerase-aktivierten Adapters, umfassend eine an ein doppelsträngiges Oligonukleotid gebundene Vaccinia-Virus-Topoisomerase I;
(b) Bereitstellen eines doppelsträngigen Zielpolynukleotids, umfassend eine Triplett-Nukleotidsequenz am 5'-Ende eines oder beider Stränge, wobei die Triplett-Nukleotidsequenz aus GGT, GGG, TGT, GTT, TGG, GGA, GTG, CGG, CGT und GGC ausgewählt ist; und
(c) Inkontaktbringen des Topoisomerase-aktivierten Adapters mit dem doppelsträngigen Zielpolynukleotid, sodass die Topoisomerase den aktivierten Adapter kovalent an die 5'-Enden des doppelsträngigen Zielpolynukleotids bindet,
wodurch ein Polynukleotid-Adapter an ein doppelsträngiges Zielpolynukleotid gebunden wird.

11. Verfahren zum Herstellen von angepassten PCR-Amplikonen, das Verfahren umfassend:
(i) Amplifizieren einer ersten Nukleinsäure unter Verwendung eines ersten Oligonukleotid-Primers und eines zweiten Oligonukleotid-Primers, um ein erstes Amplikon herzustellen, wobei mindestens einer der ersten und zweiten Primer einen 5'-Schwanz umfasst, der in einer Triplett-Nukleotidsequenz endet, die aus GGT, GGG, TGT, GTT, TGG, GGA, GTG, CGG, CGT und GGC ausgewählt ist, und Amplifizieren einer zweiten Nukleinsäure unter Verwendung eines dritten Primers und eines vierten Primers, um ein zweites Amplikon herzustellen, wobei mindestens einer der dritten und vierten Primer einen 5'-Schwanz umfasst, der in einer Triplett-Nukleotidsequenz endet, die nicht GGT, GGG, TGT, GTT, TGG, GGA, GTG, CGG, CGT oder GGC ist; und
(ii) Inkontaktbringen des ersten Amplikons und des zweiten Amplikons mit Topoisomerase-aktivierten Adaptern, umfassend eine an einen Adapter gebundene Vaccinia-Virus-Topoisomerase I, sodass die Topoisomerase einen Adapter kovalent an das 5'-Ende des ersten Amplikons und das 5'-Ende des zweiten Amplikons bindet.

12. Verfahren nach Anspruch 11, wobei:
(i) mindestens einer der ersten und zweiten Primer einen 5'-Schwanz umfasst, der in der Triplett-Nukleotidsequenz GGT oder GGG endet; und/oder
(ii) der 5'-Schwanz des mindestens einen der dritten und vierten Primer in einer Triplett-Nukleotidsequenz endet, die aus ACA, TTA, ACG, TCC, ACC, ACT, TCT, CCA, CCC, TCA, TCG, TTC, TTG, CCG und AAA ausgewählt ist, wobei optional mindestens einer der dritten und vierten Primer einen 5'-Schwanz umfasst, der in der Triplett-Nukleotidsequenz ACA endet.

13. Kit, umfassend:
- eine Vaccinia-Virus-Topoisomerase I;
- einen doppelsträngigen Oligonukleotid-Adapter, umfassend eine Topoisomerase-Zielstelle;
- ein Primerpaar, bestehend aus einem ersten und einem zweiten Oligonukleotid-Primer, wobei mindestens einer der ersten und zweiten Primer ein 5'-Schwanz-Primer ist, der eine Triplett-Nukleotidsequenz, die aus GGT, GGG, TGT, GTT, TGG, GGA, GTG, CGG, CGT und GGC ausgewählt ist, am 5'-Ende des Schwanzes aufweist.

14. Kit nach Anspruch 13, wobei:
(i) mindestens einer der ersten und zweiten Primer einen 5'-Schwanz umfasst, der in der Triplett-Nukleotidsequenz GGT oder GGG endet;
(ii) das Kit ferner ein Primerpaar umfasst, bestehend aus einem dritten Oligonukleotid-Primer und einem vierten Oligonukleotid-Primer, wobei mindestens einer der dritten und vierten Oligonukleotid-Primer ein 5'-Schwanzprimer ist, der in einer Triplett-Nukleotidsequenz endet, die nicht GGT, GGG, TGT, GTT, TGG, GGA, GTG, CGG, CGT oder GGC ist, wobei optional der 5'-Schwanz des mindestens einen der dritten und vierten Primer in einer Triplett-Nukleotidsequenz endet, die aus ACA, TTA, ACG, TCC, ACC, ACT, TCT, CCA, CCC, TCA, TCG, TTC, TTG, CCG und AAA ausgewählt ist, und wobei ferner optional mindestens einer der dritten und vierten Primer einen 5'-Schwanz umfasst, der in der Triplett-Nukleotidsequenz ACA endet.

15. Kit nach einem der Ansprüche 13 oder 14, wobei die Topoisomerase auf den Adapter geladen ist und/oder der Adapter ferner eine Click-reaktive Gruppe, ein Fluorophor, ein Konjugationsmittel, eine Pull-Down-Gruppe, eine Tethering-Gruppe, einen Marker, eine modifizierte Base, einen basischen Rest, einen Zwischenadapter, einen Amplifikations-Adapter, einen Haarnadel-Adapter, einen eindeutigen molekularen Identifikator, eine Helikase-Bindungsstelle und/oder einen Spacer umfasst.

## Revendications

1. Procédé de préparation d'une banque d'acides nucléiques, le procédé comprenant :
(a) la fourniture d'une pluralité d'adaptateurs activés par la topoisomérase, chacun comprenant une topoisomérase I du virus de la vaccine liée à un oligonucléotide double brin ;
(b) la fourniture d'une pluralité de polynucléotides cibles double brin, chacun comprenant une séquence de triplets de nucléotides à l'extrémité 5' d'un ou des deux brins, dans lequel la séquence de triplets de nucléotides est sélectionnée parmi GGT, GGG, TGT, GTT, TGG, GGA, GTG, CGG, CGT et GGC ; et
(c) la mise en contact des adaptateurs activés par la topoisomérase avec la pluralité de polynucléotides cibles double brin, de telle sorte que la topoisomérase lie de manière covalente les adaptateurs activés aux extrémités 5' des polynucléotides cibles double brin, préparant ainsi une banque d'acides nucléiques.

2. Procédé selon la revendication 1, qui comprend en outre la production de la pluralité de polynucléotides cibles double brin comprenant la séquence de triplets de nucléotides à l'extrémité 5' d'un ou des deux brins par ACP à l'aide d'une première amorce oligonucléotidique et d'une deuxième amorce oligonucléotidique, dans lequel au moins l'une parmi les première et deuxième amorces comprend une queue 5' qui se termine par la séquence de triplets de nucléotides.

3. Procédé selon la revendication 2, dans lequel les première et deuxième amorces oligonucléotidiques comprennent toutes deux une queue 5' qui se termine par la séquence de triplets de nucléotides.

4. Procédé selon l'une quelconque des revendications 1 à 3, dans lequel les adaptateurs sont des adaptateurs de séquençage.

5. Procédé selon l'une quelconque des revendications 1 à 4, dans lequel la séquence de triplets de nucléotides est GGT ou GGG.

6. Procédé selon l'une quelconque des revendications 1 à 5, dans lequel la séquence de triplets de nucléotides à l'extrémité 5' d'un ou des deux brins de la pluralité de polynucléotides cibles double brin est immédiatement en 5' d'une deuxième séquence de triplets de nucléotides sélectionnée parmi GGT, GGG, TGT, GTT, TGG, GGA, GTG, CGG, CGT et GGC, éventuellement dans lequel la deuxième séquence de triplets de nucléotides à l'extrémité 5' d'un ou des deux brins de la pluralité de polynucléotides cibles double brin est immédiatement en 5' d'une troisième séquence de triplets de nucléotides sélectionnée parmi GGT, GGG, TGT, GTT, TGG, GGA, GTG, CGG, CGT et GGC.

7. Procédé selon l'une quelconque des revendications 1 à 6, dans lequel :
i. les polynucléotides cibles à double brin ont des extrémités franches ; ou
ii. les polynucléotides cibles double brin comprennent un surplomb nucléotidique, éventuellement dans lequel les polynucléotides cibles double brin sont à queue A.

8. Procédé selon l'une quelconque des revendications 1 à 7, dans lequel l'adaptateur comprend en outre un groupe réactif de la chimie click, un fluorophore, un agent de conjugaison, un groupe de capture, un fragment d'ancrage, un marqueur, une base modifiée, un résidu basique, un adaptateur intermédiaire, un adaptateur d'amplification, un adaptateur en épingle, un identifiant moléculaire unique, un site de liaison à l'hélicase et/ou un espaceur.

9. Procédé selon l'une quelconque des revendications 1 à 8, comprenant en outre la détermination de la présence, de l'absence ou d'une ou plusieurs caractéristiques des polynucléotides cibles double brin liés de manière covalente aux adaptateurs, le procédé comprenant :
(a) la mise en contact du polynucléotide cible avec un nanopore ou un mutant de celui-ci de telle sorte que le polynucléotide cible se déplace par rapport au pore ; et
(b) la prise d'une ou plusieurs mesures lorsque le polynucléotide se déplace par rapport au pore,
déterminant ainsi la présence, l'absence ou une ou plusieurs caractéristiques du polynucléotide.

10. Procédé de fixation d'un adaptateur polynucléotidique à un polynucléotide cible double brin, le procédé comprenant :
(a) la fourniture d'un adaptateur activé par la topoisomérase comprenant une topoisomérase I du virus de la vaccine liée à un oligonucléotide double brin ;
(b) la fourniture d'un polynucléotide cible double brin comprenant une séquence de triplets de nucléotides à l'extrémité 5' d'un ou des deux brins, dans lequel la séquence de triplets de nucléotides est sélectionnée parmi GGT, GGG, TGT, GTT, TGG, GGA, GTG, CGG, CGT et GGC ; et
(c) la mise en contact de l'adaptateur activé par la topoisomérase avec le polynucléotide cible double brin de telle sorte que la topoisomérase lie de manière covalente l'adaptateur activé aux extrémités 5' du polynucléotide cible double brin,
fixant ainsi un adaptateur polynucléotidique à un polynucléotide cible double brin.

11. Procédé de production d'amplicons ACP adaptés, le procédé comprenant :
(i) l'amplification d'un premier acide nucléique à l'aide d'une première amorce oligonucléotidique et d'une deuxième amorce oligonucléotidique pour produire un premier amplicon, dans lequel au moins l'une parmi les première et deuxième amorces comprend une queue 5' qui se termine par une séquence de triplets de nucléotides sélectionnée parmi GGT, GGG, TGT, GTT, TGG, GGA, GTG, CGG, CGT et GGC et l'amplification d'un second acide nucléique à l'aide d'une troisième amorce et d'une quatrième amorce pour produire un second amplicon, dans lequel au moins l'une parmi les troisième et quatrième amorces comprend une queue 5' qui se termine par une séquence de triplets de nucléotides qui n'est pas GGT, GGG, TGT, GTT, TGG, GGA, GTG, CGG, CGT ou GGC ; et
(ii) la mise en contact du premier amplicon et du second amplicon avec des adaptateurs activés par la topoisomérase comprenant une topoisomérase I du virus de la vaccine liée à un adaptateur de telle sorte que la topoisomérase lie de manière covalente un adaptateur à l'extrémité 5' du premier amplicon et à l'extrémité 5' du second amplicon.

12. Procédé selon la revendication 11, dans lequel :
(i) au moins l'une parmi les première et deuxième amorces comprend une queue 5' qui se termine par la séquence de triplets de nucléotides GGT ou GGG ; et/ou
(ii) la queue 5' de l'au moins l'une parmi les troisième et quatrième amorces se termine par une séquence de triplets de nucléotides sélectionnée parmi ACA, TTA, ACG, TCC, ACC, ACT, TCT, CCA, CCC, TCA, TCG, TTC, TTG, CCG et AAA ; éventuellement, au moins l'une parmi les troisième et quatrième amorces comprend une queue 5' qui se termine par la séquence de triplets de nucléotides ACA.

13. Kit comprenant :
- une topoisomérase I du virus de la vaccine ;
- un adaptateur oligonucléotidique double brin comprenant un site cible de topoisomérase ;
- une paire d'amorces constituée d'une première et d'une deuxième amorces oligonucléotidiques, dans lequel au moins l'une parmi les première et deuxième amorces est une amorce à queue 5' ayant une séquence de triplets de nucléotides sélectionnée parmi GGT, GGG, TGT, GTT, TGG, GGA, GTG, CGG, CGT et GGC à l'extrémité 5' de la queue.

14. Kit selon la revendication 13, dans lequel :
(i) au moins l'une parmi les première et deuxième amorces comprend une queue 5' qui se termine par la séquence de triplets de nucléotides GGT ou GGG ;
(ii) le kit comprend en outre une paire d'amorces constituée d'une troisième amorce oligonucléotidique et d'une quatrième amorce oligonucléotidique, dans lequel au moins l'une parmi les troisième et quatrième amorces oligonucléotidiques est une amorce à queue 5' qui se termine par une séquence de triplets de nucléotides qui n'est pas GGT, GGG, TGT, GTT, TGG, GGA, GTG, CGG, CGT ou GGC, éventuellement dans lequel la queue 5' de l'au moins une parmi les troisième et quatrième amorces se termine par une séquence de triplets de nucléotides sélectionnée parmi ACA, TTA, ACG, TCC, ACC, ACT, TCT, CCA, CCC, TCA, TCG, TTC, TTG, CCG et AAA, et en outre éventuellement dans lequel au moins l'une parmi les troisième et quatrième amorces comprend une queue 5' qui se termine par la séquence de triplets de nucléotides ACA.

15. Kit selon l'une quelconque de la revendication 13 ou de la revendication 14, dans lequel la topoisomérase est chargée sur l'adaptateur et/ou l'adaptateur comprend en outre un groupe réactif de la chimie click, un fluorophore, un agent de conjugaison, un groupe de capture, un fragment d'ancrage, un marqueur, une base modifiée, un résidu basique, un adaptateur intermédiaire, un adaptateur d'amplification, un adaptateur en épingle, un identifiant moléculaire unique, un site de liaison à l'hélicase et/ou un espaceur.
